# EUROPEAN PATENT SPECIFICATION

(11) **EP 1 188 058 B1**
(45) Date of publication and mention of the grant of the patent: **11.05.2005**
(21) Application number: 00946608.7
(22) Date of filing: 19.06.2000
(51) Int. Cl.: G01N 33/543, G06F 19/00

(54) **METHOD AND APPARATUS FOR ASSAYING A DRUG CANDIDATE TO ESTIMATE A PHARMACOKINETIC PARAMETER ASSOCIATED THEREWITH**
Verfahren und Vorrichtung zur Untersuchung von Wirksstoffskandidaten und zur Bestimmung ihrer pharmakokinetischen Parametern
PROCEDE ET APPAREIL DE DOSAGE BIOLOGIQUE D'UNE DROGUE CANDIDATE DESTINES A EVALUER UN PARAMETRE PHARMACOCINETIQUE ASSOCIE

(30) Priority: 18.06.1999 US 336865
(43) Date of publication of application: 20.03.2002
(73) Proprietor: Biacore AB, 754 50 Uppsala (SE)
(72) Inventor: HÄMÄLÄINEN, Maikku, S-755 97 Uppsala (SE); KARLSSON, Robert, S-754 40 Uppsala (SE); LÖFAS, Stefan, S-756 46 Uppsala (SE)
(74) Representative: Widén, Björn
(86) International application number: PCT/SE2000/001297
(87) International publication number: WO 2000/079268

(56) References cited:
- WO-A1-99/63333
- US-A- 5 313 264
- US-A- 5 492 840
- 87th Annual Meeting of the American Association for Cancer Research, Washinton, DC, April 20-24, 1996, Giorgio, N. et al: "Pharmacokinetics in a phase I study of a chimerized monoclonal-epidermal growth factor receptor (EGFr) antibody C225, as determined by surface plasmon resonance", No. 1230, see abstract, XP002901450
- BIOSENSORS & BIOELECTRONICS, Volume 9, 1994, Ingemar Lundström, "Real-time biospecific interaction analysis" page 725 - page 736, XP002901451,

## Description

### TECHNICAL FIELD

This invention is generally directed to a method and apparatus for assaying a drug candidate and, more specifically, to a method for measuring the binding interaction between a drug candidate and sensing surface-bound biomolecules of a biosensor to determine a binding interaction parameter of the drug candidate, and then comparing the binding interaction parameter against a predetermined drug correlation graph (*e*.*g*., a mathematical expression) to estimate at least one pharmacokinetic parameter.

### BACKGROUND OF THE INVENTION

A variety of experimental techniques are currently used to determine chemical, physical and biological properties associated with low molecular weight substances, particularly in the context of drug discovery. For example, researchers are often concerned with determining a variety of chemical, physical and biological properties associated with drug candidates for screening purposes. The determination of such properties often plays a pivotal role in the drug development and screening process.

More specifically, it has long been recognized that the intensity and duration of the pharmacological effect of a systemically acting drug are functions not only of the intrinsic activity of the drug, but also of its absorption, distribution, metabolism, and excretion (ADME) characteristics within the human body. These so-called ADME characteristics are all intimately related to the scientific discipline known as "pharmacokinetics." Pharmacokinetics is commonly referred to as the study of the time courses (*i.e*., kinetics) associated with the dynamic processes of ADME of a drug and/or its metabolites within a living organism, and is closely interrelated with the fields of biopharmaceutics, pharmacology, and therapeutics.

Because the body delays the transport of drug molecules across membranes, dilutes them into various compartments of distribution, transforms them into metabolites, and eventually excretes them, it is often difficult to accurately predict the pharmacological effect of promising new drug candidates. Researchers, however, commonly use pharmacokinetic ADME studies as one method to predict the efficacy of a drug at a site of action within the body.

Traditionally, researchers involved with pre-clinical ADME studies have used pharmacokinetic/mathematical models coupled with actual drug concentration data from blood (or serum or plasma) and/or urine, as well as concentration data from various tissues, to characterize the behavior and "fate" of a drug within living organisms. As is appreciated by those skilled in the art, the mathematical equations associated with pharmacokinetics are generally based on models that conceive the body as a multicompartmental organism. In such models it is presumed that the drug and/or its metabolites are equitably dispersed in one or several fluids/tissues of the organism. Any conglomerate of fluid or tissue which acts as if it is kinetically homogeneous may be termed a "compartment." Each compartment acts as an isotropic fluid in which the molecules of drug that enter are homogeneously dispersed and where kinetic dependencies of the dynamic pharmacokinetic processes may be formulated as functions of the amounts or concentrations of drug and metabolites therein. Stated somewhat differently, the conceptual compartments of the body are separated by barriers that prevent the free diffusion of drug among them; the barriers are kinetically definable in that the rate of transport of drug or metabolite across membrane barriers between compartments is a function of, for example, the amounts or concentrations of drug and metabolites in the compartments, the permeability of various membranes, and/or the amount of plasma protein binding and general tissue binding.

More specifically, pharmacokinetic/mathematical models are commonly used by pharmacokineticists to represent drug absorption, distribution, metabolism, and excretion as functions of time within the various tissues and organs of the body. In such models, the movement of the administered drug throughout the body is concisely described in mathematical terms (*e*.*g*., a set of differential equations). The predictive capability of such models lies in the proper selection and development of mathematical functions that parameterize the essential factors governing the kinetic process under consideration.

For example, a drug that is administered by intravenous injection may be assumed to distribute rapidly in the bloodstream. A pharmacokinetic/mathematical model that describes this situation may be a tank containing a volume of fluid that is rapidly equilibrated with the drug. Because a fraction of the drug in the body is continually eliminated as a function of time (*e*.*g*., excreted by the kidneys and metabolized by the liver), the concentration of the drug in the hypothetical tank may be characterized by two parameters: (1) the volume of fluid in the tank that will dilute the drug, and (2) the elimination rate of the drug per unit of time, both of which are generally considered to be constant Thus, if a known set of drug concentrations in the tank is determined at various time intervals by, for example, sampling, then the volume of fluid in the tank and rate of drug elimination may be estimated. This information may then, in turn, be used for predicting the disposition of the drug within a human body.

Theoretically, an unlimited number of models may be constructed to describe the kinetic processes of drug absorption, distribution, metabolism, and excretion within the various tissues and organs of the human body. In general, however, the number of useful models is limited due to practical considerations associated with blood, tissue and/or organ sampling. As a result, and as is appreciated by those skilled in the art, two major types of models have been developed by pharmacokineticists: (1) compartmental models; and (2) physiologic models.

In pharmacokinetic compartmental models, the body is represented as a series of compartments that communicate reversibly with each other. Each compartment is not a real physiological or anatomic region; rather, each compartment is considered to be inclusive of all tissues that have similar blood flow and drug affinity. For example, a compartmental model may consist of one or more peripheral compartments representing tissue(s) connected to a central compartment representing the blood stream. Conceptually, the drug moves dynamically into and out of the central compartment and into and out of each of the peripheral compartments. As such, rate constants may be used to represent the overall rate process for the drug's disposition within each compartment. Compartment models are generally based on linear assumptions using linear differential equations, and are particularly useful when there is little information known about the tissues and their respective drug concentrations.

In contrast, pharmacokinetic physiologic models are based on known anatomic and physiologic data, data which is kinetically described in view of the actual blood flow volumes responsible for distributing the drug to the various parts of the body. Because there are many tissue organs in the body, each tissue volume must be estimated and its drug concentration and rate of change described mathematically (tissues having similar blood perfusion properties, however, are typically grouped together). Unfortunately, much of the information required to adequately describe such pharmacokinetic physiologic models are often very difficult to obtain experimentally. Nevertheless, such physiologically based models are commonly used in conjunction with animal data and interspecies scaling techniques to predict the drug's disposition within a human body.

More importantly, however, is that pharmacokinetic/mathematical models, and knowledge of their associated ADME parameters play an extremely important role in drug discovery and development. A typical example is a drug that is active following intravenous administration but is considerably less active after comparable oral doses. Having appropriate pharmacokinetic information may reveal (1) whether the drug was poorly absorbed to yield sub-therapeutic circulating levels, or (2) whether the drug experienced pre-systemic metabolism to an inactive metabolite. Such information may also provide guidance for subsequent decisions, such as (1) whether to improve drug absorption by altering the salt form or formulation, (2) whether to investigate the possibility of making prodrugs, or (3) whether to consider a parenteral route of administration.

In addition to the foregoing, pharmacokinetic/mathematical models are also generally considered extremely useful for, among other things: (1) predicting plasma, tissue, and urine drug levels with any dosage regimen; (2) calculating the optimum dosage regimen for an individual patient; (3) estimating the possible accumulation of drugs and/or metabolites; (4) correlating drug concentrations with pharmacologic and toxicologic activity (*i*.*e*., pharmacodynamics); (5) evaluating differences in the rate or extent of availability between formulations (*i*.*e*., bioequivalence); (6) describing how changes in physiology or disease affect the absorption, distribution, and/or elimination of the drug; and (7) explaining drug-drug and food-drug interactions.

Lastly, pharmacokinetic ADME data has also become an integral part of the pharmacological characterization process of promising new drug candidates. Regulatory agencies, such as the U.S. Food and Drug Administration, now require (1) a determination of pharmacokinetic ADME data in Phase I drug studies, and (2) a submission of pharmacokinetic ADME data as part of a New Drug Application. In this context, such pharmacokinetic ADME data is deemed essential for predicting the behavior and fate of the drug candidate within the human body.

Accordingly, there is a need in the art for improved methods for determining one or more pharmacokinetic parameters associated with absorption, distribution, metabolism, and excretion of a drug candidate. There is also a need for apparatuses useful for carrying out such methods. The present invention fulfills these needs and provides further related advantages.

WO 99/63333 (published after the priority date of the present application and therefore not relevant to inventive step according to Article 54(3) EPC) discloses the characterization of an analyte and/or ligand by determining kinetic parameters for the biospecific interaction of the analyte with a biosensor surface-bound ligand in several different aqueous characterizing solutions, such as acidic, basic, ionic, organic, detergent or chelating solutions. By comparing the characterization of the ligand and/or analyte with a set of similar characterizations made for one or more predetermined test molecules, the nature of the binding activity of the analyte or ligand may be predicted.

Giorgio, N., et al. (1966) in the 87^{th} Annual Meeting of the American Association for Cancer Research, Washington, April 20-24, abstract 1230 discloses the use of surface plasmon resonance (SPR) performed via Biacore™ technology to determine circulating levels of C225, a human/murine chimeric monoclonal antibodythat binds with high affinity to the EGF receptor. SPR was also used to determine anti-C225 antibody concentrations.

### SUMMARY OF THE INVENTION

In brief, the present invention is directed to a method and apparatus for assaying a drug candidate. More specifically, this invention discloses a method for measuring the binding interaction between a drug candidate and sensing surface-bound biomolecules of a biosensor to determine a binding interaction parameter of the drug candidate, and then comparing the binding interaction parameter against a predetermined drug correlation graph to estimate at least one pharmacokinetic parameter of the drug candidate. The at least one pharmacokinetic parameter may, for example, be one or more of ADME.

In another embodiment of the present invention, at least two pharmacokinetic parameters of the drug candidate are determined, and in yet another embodiment, at least one pharmacokinetic parameter and a solubility property of the drug candidate are determined. Such pharmacokinetic parameters and/or solubility property may be determined when the one or more sensing surface-bound biomolecules are selected from, for example, liposomes, plasma proteins, CYP 450 enzymes, metabolic enzymes, or transport proteins.

The biosensor used in the practice of the present invention may utilize a mass-sensing technique, such as surface plasmon resonance. In addition, the biosensor may further employ a sensor chip, wherein the sensor chip comprises a free electron metal that includes a sensor surface, wherein the free electron metal is copper, silver, aluminum or gold. The sensor chip may further comprise a hydrogel coupled to the sensor surface, wherein the hydrogel has a plurality of functional groups, and wherein the one or more sensing surface-bound biomolecules are covalently bonded to the hydrogel.

In a more specific embodiment, a sensor surface adopted for use with a biosensor is disclosed. The sensor surface comprises a hydrogel matrix coating coupled to a top surface of the sensor surface, wherein the hydrogel matrix coating has plurality of functional groups. At least two different liposomes are bonded to the plurality of functional groups at discrete and noncontiguous locations on the hydrogel matrix coating of the sensor surface. In one embodiment, the sensor surface is a sensor chip, and a free electron metal is interposed between the hydrogel matrix and the top surface of the sensor surface.

In another embodiment of this invention, an apparatus is disclosed for assaying a drug candidate, wherein the apparatus comprises a biosensor having one or more sensing surface-bound biomolecules associated therewith and capable of measuring at least one binding interaction parameter of the drug candidate, and a computer memory containing a data structure for comparing the at least one binding interaction parameter against at least one mathematical expression correlated from binding interaction data associated with known drug compounds having known pharmacokinetic parameters to determine an estimate of at least one pharmacokinetic parameter of the drug candidate.

These and other aspects of the present invention will be evident upon reference to the following detailed description and related Figures.

### BRIEF DESCRIPTION OF THE DRAWINGS

Figure 1A illustrates a sensorgram reflective of the steady-state binding levels associated with a selected drug-biomolecule interaction.
Figure 1B illustrates an enlarged portion of the semsorgram of Figure 1A and shows sensorgram irregularities.
Figure 1C illustrates an enlarged portion of the sensorgram of Figure 1A, wherein bulk-refractive index effects have been eliminated.
Figure 2A illustrates a single "dip" depicting the reflected light intensity associated with a homogeneous sensor surface.
Figure 2B illustrates a number of "dips" depicting the non-averaged reflected light intensities associated with a non-homogeneous sensor surface.
Figure 2C illustrates a broadening of the "dip" depicting the averaged reflected light intensities associated with a non-homogeneous sensor surface.
Figure 3 shows a high-level block diagram of an exemplary computer system for assaying a drug candidate in accordance with the methods of the present invention.
Figure 4 depicts a correlation graph having known KD values for known compounds plotted along the abscissa (*i.e*., the x-axis) and corresponding measured KD values obtained via the biosensor plotted along the ordinate (*i*.*e*., the y-axis) for nine drugs with known levels of plasma protein binding
Figure 5 depicts a correlation graph having respective binding levels at 100 µM (R 100 µM) divided by the molecular weight of known drug compounds' plotted along the abscissa (*i.e*., the x-axis) and corresponding human serum albumin binding percentage, as measured by equilibrium dialysis, plotted along the ordinate (*i.e*., the y-axis).
Figure 6 depicts reference subtracted sensorgram traces for each of three drug candidates A-C.
Figure 7 depicts a correlation graph having known fraction absorbed in humans (FA%) plotted along the ordinate (*i*.*e*., the y-axis) and corresponding calibrated (*i*.*e*., reference subtracted) steady state binding levels for each drug at 500 µM plotted along the abscissa in a 10-logarithm scale (*i*.*e*., the x-axis).
Figure 8 depicts a general principal for interaction analysis. A sensor chip surface is placed in contact with the IFC (Integrated Fluidic Cartridge) and the detection unit. Continuous buffer flows through the IFC and over the surface. Samples are injected over the surface, using the autoinjector, and refractive index changes are detected by the detection unit.

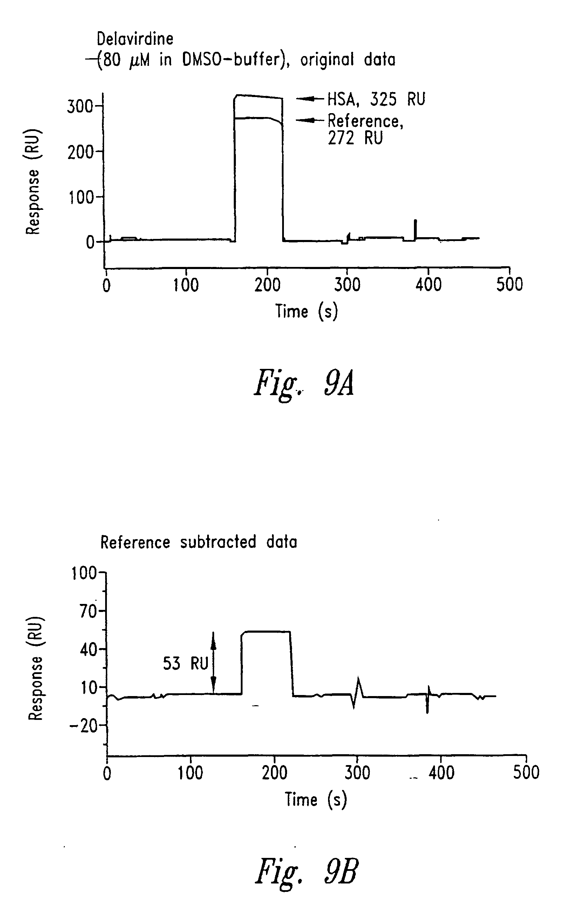

Figures 9a-9d depict the following: (a) Injection of delavirdine over reference and HSA flowcell. A high response level was obtained since the DMSO concentration of sample and assay buffer was not exactly matched in sample preparation. (b) Reference data subtracted from HSA data. (c) A series of 8 calibration solutions (running buffer with different DMSO concentrations) were run at the beginning and end of each experiment. The responses of the calibration solutions, obtained from the reference surface, covered a range from -500 to +1000 RU relative to the baseline. A DMSO calibration curve was created by plotting difference in response between HSA and reference flowcell (DMSO correction factor) versus response in reference flowcell. (d) Compound responses were corrected for DMSO bulk differences via the calibration curves.
Figure 10 illustrates a dose-response curves of six compounds binding to HSA. In (a) and (b) warfarin and naproxen displays mono-phasic binding curves obtained by fitting data to a steady state model. In (c) and (d) binding of digitoxin and ritonavir appears to be biphasic. Quinine and rifampicin give linear binding curves, (e) and (f). Curves in (c-f) were obtained by four-parameter fits and do not represent K_{D} fits.
Figure 11 depicts a biosensor data of compounds binding to HSA correlated to data obtained with other techniques, references in Table 7. (Literature data for each compound varies depending upon experimental conditions).
Figure 12 illustrates a ranking of binding levels of 19 compounds. The same set of compounds were ranked on the same sensor surface, in duplicate, on the day of HSA immobilization (shown) and in duplicate a week later (not shown). Diazepam and warfarin were used as marker compounds to group HSA binding levels into Low, Medium or High. Warfarin was injected early, in the middle and late in each assay as a control of the HSA binding activity. L, M and H represent the classification into Low, Medium or High level binders based on reported literature levels of % bound.

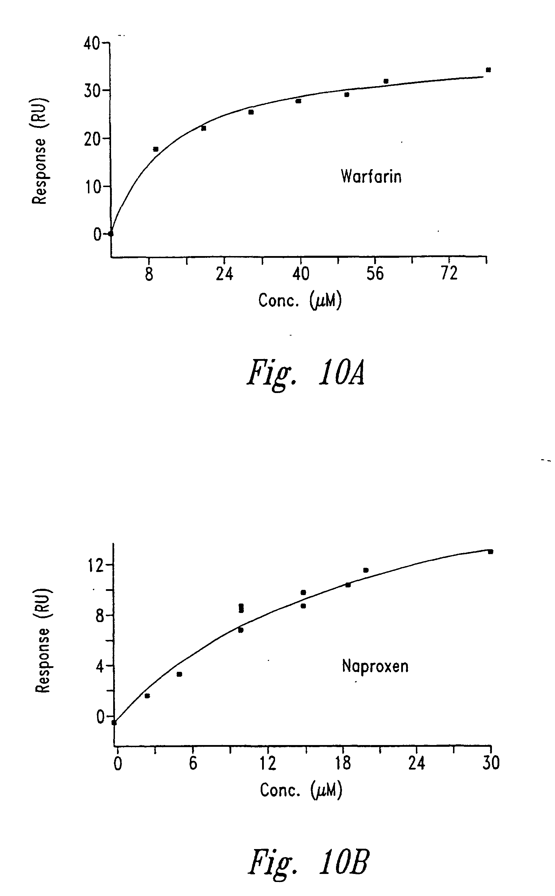

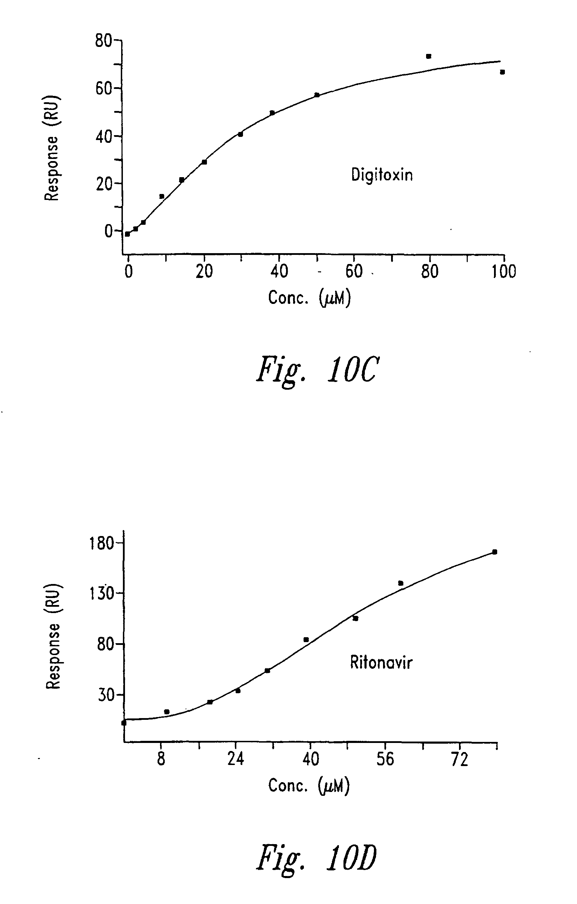

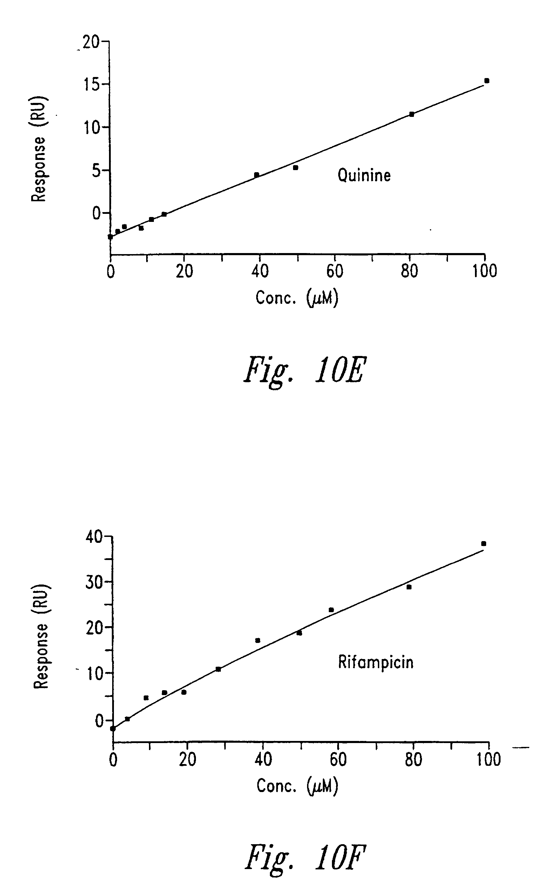

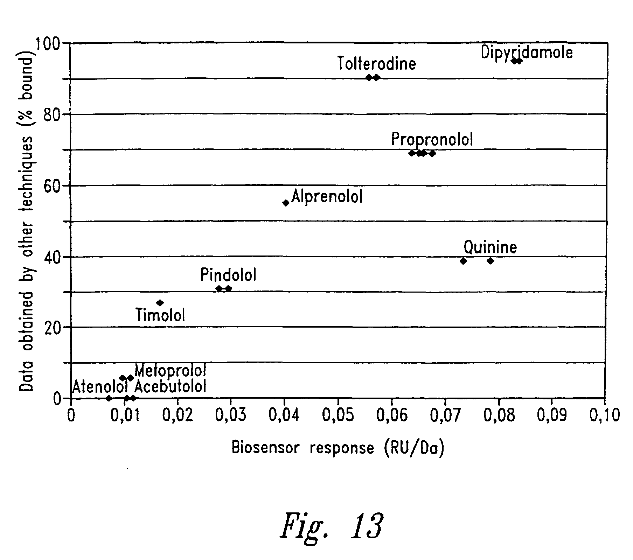

Figure 13 illustrates the biosensor data of AGP binding correlated to data obtained with other techniques, references last in Discussion. Prior to immobilisation AGP (Sigma G-9885) was modified with PDEA (2-(2-pyridinyldithio)ethaneamine hydrocloride from Biacore). AGP (200 ug/ml in 10 mM Na-citrate pH 3.6) was then immobilised to a level of 7000 RU using thiol coupling (Löfås et al., "Methods for Site Controlled Coupling to Carboxymethyldextran Surfaces in Surface Plasmon Resonance Sensors," *Biosensors & Bioelectronics, 10*:813-822 (1995)). All other experimental conditions were the same as for the HSA correlation study in Figure 10.
Figure 14 depicts CYP2D6 and CYP2C19 immobilized in flow cells 3 and 2 to 13000 and 11000 RU, respectively.
Figure 15 depicts CYP2E1 and CYP3A4 immobilized in flow cells 2 and 3 to 8500 and 7300 RU, respectively.

### DETAILED DESCRIPTION OF THE INVENTION

The present invention is directed to a method for assaying a drug candidate and, more specifically, to a method and apparatus for measuring the binding interaction between at least one drug candidate and sensing surface-bound biomolecules of a biosensor to determine a binding interaction parameter of the drug candidate, and then comparing the binding interaction parameter against a predetermined drug correlation graph (*e*.*g*., a mathematical expression fitted to a series of known data points) to determine an estimate of at least one pharmacokinetic parameter. Although many specific details of certain embodiments of the invention are set forth in the following detailed description and accompanying Figures, those skilled in the art will recognize that the present invention may have additional embodiments, or that the invention may be practiced without several of the details described herein.

For purposes of clarity and to assist in understanding the full scope of the present invention, a brief review of the nomenclature associated with pharmacokinetics has been provided. As used within the context of the present invention, the following pharmacokinetic terms shall be construed broadly, and shall have their generally accepted meanings as set forth below. (As previously noted, "pharmacokinetics" refers to the study of the kinetics associated with the dynamic processes of absorption, distribution, metabolism, and excretion (ADME) of a drug and/or its metabolites within a living organism.)

"Absorption" refers to the process of uptake of a drug compound from the site of administration into the systemic circulation. The transfer of drug across the intestinal lumen is generally referred to as oral absorption, whereas the transfer of drug across an external physiological barrier is referred to general absorption. As disclosed herein, the pharmacokinetic parameter of absorption may be estimated from biosensor data associated with a sensor chip having, for example, a plurality of appropriate liposomes immobilized thereon.

"Distribution" refers to the transfer of a drug compound from the site of administration to the total systemic circulation and then to extracellular and intracellular water and tissues. Drug distribution is usually a rapid and reversible process. As disclosed herein, the pharmacokinetic parameter of distribution may be estimated from biosensor data associated with a sensor chip having, for example, a plurality of appropriate plasma proteins, liposomes, and/or transport proteins immobilized thereon.

"Metabolism" refers to the sum of all the chemical reactions for biotransformation of endogenous and exogenous substances which take place in the living cell. As disclosed herein, the pharmacokinetic parameter of metabolism may be estimated from biosensor data associated with a sensor chip having, for example, a plurality of appropriate metabolic enzymes immobilized thereon.

"Excretion" refers to the final elimination or loss of a drug from the body. Drug excretion includes both passive diffusion and relative specific carrier mediated excretion. Drugs may be excreted, unchanged or as metabolites, in urine via the kidneys or in feces via the bile and/or the intestine. Volatile compounds are often excreted in expired air by the lungs. As disclosed herein, the pharmacokinetic parameter of excretion may be estimated from biosensor data associated with a sensor chip having immobilized thereon, for example, an antibody that specifically detects the drug, as well as other proteins/receptors having a high affinity/specificity against the drug candidate. Such antibodies and proteins/receptors may be used to quantify the concentration/amount of the drug in different body fluids (*e*.*g*., urine/feces) and tissues, using a direct binding assay.

In addition to these ADME parameters, the solubility of a drug is also an important property that may be measured by the methods of the present invention. "Solubility" refers to the ability of two substances to form a homogeneous solution or mixture with each other. Solubility is important for the dissolution of drug given in solid dosage form. As disclosed herein, the solubility of a drug candidate may be estimated from sensorgram irregularities associated with reflectance minimum and dip-shape of biosensor data.

Furthermore, and as used herein, the term "parameter" refers to a constant or variable term in a function (*e*.*g*., a mathematical expression) that determines the specific form of the function but not necessarily its general nature. For example, the constant term "*a*" in the function *f(x)* = *ax*, where "*a*" determines only the slope of the line described by *f(x)*, is referred to as a parameter. As such, the term "binding interaction parameter" refers to those constant or variable terms that are related to the binding interaction between a drug candidate and a sensing surface-bound biomolecule and includes, for example, association and dissociation rate constants, as well as maximum binding capacity. Similarly, the term "pharmacokinetic parameter" refers to those constant and variable terms that are related to the disposition of the drug candidate within a living organism and includes, for example: volume of distribution; total clearance; protein binding; tissue binding; metabolic clearance; renal clearance; hepatic clearance; biliary clearance; intestinal absorption; bioavailability; relative bioavailability; intrinsic clearance; mean residence time; maximum rate of metabolism; Michaelis-Menten constant; partitioning coefficients between tissues and blood (or plasma) such as those partitioning coefficients associated with the blood brain barrier, blood placenta barrier, blood human milk partitioning, blood adipose tissue partitioning, and blood muscle partitioning; fraction excreted unchanged in urine; fraction of drug systemically converted to metabolites; elimination rate constant; half-life; and secretion clearance.

The methods of the present invention are intended to be carried out by use of an affinity-based biosensor. As is appreciated by those skilled in the art, "biosensors" are analytical devices for analyzing minute quantities of sample solution having an analyte of interest, wherein the analyte is analyzed by a detection device that may employ a variety of detection methods. Typically, such methods include, but are not limited to, mass detection methods, such as piezoelectric, optical, thermo-optical and surface acoustic wave (SAW) device methods, and electrochemical methods, such as potentiometric, conductometric, amperometric and capacitance methods. With regard to optical detection methods, representative methods include those that detect mass surface concentration, such as reflection-optical methods, including both internal and external reflection methods, angle, wavelength or phase resolved, for example, ellipsometry and evanescent wave spectroscopy (EWS), the latter including surface plasmon resonance (SPR) spectroscopy, Brewster angle refractometry, critical angle refractometry, frustrated total reflection (FTR), evanescent wave ellipsometry, scattered total internal reflection (STIR), optical wave guide sensors, evanescent wave-based imaging such as critical angle resolved imaging, Brewster angle resolved imaging, SPR angle resolved imaging, and the like. Further, photometric methods based on, for example, evanescent fluorescence (TIRF) and phosphorescence may also be employed, as well as waveguide interferometers.

In the detailed description and Examples that follow, the present invention is illustrated in the context of SPR spectroscopy. However, it is to be understood that the present invention is not limited to this detection method. Rather, any affinity-based detection method where an analyte binds to a ligand immobilized on a sensing surface may be employed, provided that a change in a property of the sensing surface is measured and quantitatively indicative of binding of the analyte to the immobilized ligand thereon. In the context of SPR spectroscopy, one exemplary class of biosensors is sold by Biacore AB (Uppsala, Sweden) under the trade name BIACORE® (hereinafter referred to as "the BIACORE instrument"). Such biosensors utilize a SPR based mass-sensing technique to provide a "real-time" binding interaction analysis between a surface bound ligand and an analyte of interest.

The BIACORE instrument includes a light emitting diode, a sensor chip covered with a thin gold film, an integrated fluid cartridge and a photo detector. Incoming light from the diode is reflected in the gold film and detected by the photo detector. At a certain angle of incidence ("the SPR angle"), a surface plasmon wave is set up in the gold layer, which is detected as an intensity loss or "dip" in the reflected light. More particularly, and as is appreciated by those skilled in the art, the phenomenon of surface plasmon resonance (SPR) associated with the BIACORE instrument is dependent on the resonant coupling of light, incident on a thin metal film, to oscillations of the conducting electrons, called plasmons, at the metal film surface. These oscillations give rise to an evanescent field which extends from the surface into the sample solution. When resonance occurs, the reflected light intensity drops at a sharply defined angle of incidence, the SPR angle, which is dependent on the refractive index within the reach of the evanescent field in the proximity of the metal surface.

Stated somewhat differently, surface plasmon resonance is an optical phenomenon arising in connection with total internal reflection of light at a metal film-liquid interface. Normally, light traveling through an optically denser medium, *e*.*g*., a glass prism, is totally reflected back into the prism when reaching an interface of an optically less dense medium, *e*.*g*., a buffer, provided that the angle of incidence is larger than the critical angle. This is known as total internal reflection. Although the light is totally reflected, a component of the incident light momentum called the evanescent wave penetrates a distance of the order of one wavelength into the buffer. The evanescent wave may be used to excite molecules close to the interface. If the light is monochromatic and *p*-polarized, and the interface between the media is coated with a thin (a fraction of the light wave-length) metal film, the evanescent wave under certain conditions will interact with free oscillating electrons (plasmons) in the metal film surface. When surface plasmon resonance occurs, light energy is lost to the metal film and the reflected light intensity is thus decreased.

The resonance phenomenon will only occur for light incident at a sharply defined angle which, when all else is kept constant, is dependent on the refractive index in the flowing buffer close to the surface. Changes in the refractive index out to about 1 µm from the metal film surface can thus be followed by continuous monitoring of the resonance angle. A detection volume is defined by the size of the illuminated area at the interface and the penetration depth of the evanescent field. It should be noted that no light passes through the detection volume (the optical device on one side of the metal film detects changes in the refractive index in the medium on the other side).

As noted above, the SPR angle depends on the refractive index of the medium close to the gold layer. In the BIACORE instrument, dextran is typically coupled to the gold surface, with the ligand being bound to the surface of the dextran layer. (Note a detailed discussion of matrix coatings for biosensor sensing surfaces is provided in U.S. Patent No. ,436,161) The analyte of interest is injected in solution form onto the sensor surface through the fluid cartridge. Because the refractive index in the proximity of the gold film depends upon (1) the refractive index of the solution (which is constant) and, (2) the amount of material bound to the surface, the binding interaction between the bound ligand and analyte can be monitored as a function of the change in SPR angle.

A typical output from the BIACORE instrument is a "sensorgram," which is a plot of response (measured in "resonance units" or "RU") as a function of time.

An increase of 1,000 RU corresponds to an increase of mass on the sensor surface of approximately 1 ng/mm². As a sample containing the analyte contacts the sensor surface, the ligand bound to the sensor surface interacts with the analyte in a step referred to as "association." This step is indicated on the sensorgram by an increase in RU as the sample is initially brought into contact with the sensor surface. Conversely, "dissociation" normally occurs when sample flow is replaced by, for example, a buffer flow. This step is indicted on the sensorgram by a drop in RU over time as analyte dissociates from the surface-bound ligand. A detailed discussion of the technical aspects of the BIACORE instrument and the phenomenon of SPR may be found in U.S. Patent No. 5,313,264.

In addition, a detailed discussion of the technical aspects of the biosensor sensor chips used in connection with the BIACORE instrument may be found in U.S. Patent No. 5,492,840. This patent discloses, among other things, that each sensor chip may have a plurality of sensing surfaces, and that such sensing surfaces may be arranged in series or parallel with respect to the fluid sample pathway of the fluid cartridge. This patent also discloses that each of the plurality of sensing surfaces of a single sensor chip may have bound thereto a unique type of ligand that is capable of interacting with an analyte in its own characteristic way.

For example, and as disclosed herein, each of the four discrete sensing surfaces of the BIACORE instrument may have immobilized thereon biomolecules such as liposomes, plasma proteins, CYP 450 enzymes, other metabolic enzymes, and/or transport/efflux proteins. By immobilizing one or a selected combination of at least two different liposomes, plasma proteins, CYP 450 enzymes, other metabolic enzymes, and/or transport/efflux proteins, onto the one or more discrete sensing surfaces of a sensor chip, one or more pharmacokinetic parameters associated with a drug candidate may be readily determined. More specifically, and in one embodiment of the present invention, it has been discovered that (1) an estimate of an "absorption" parameter of a drug candidate may be determined from the binding interactions between the drug candidate and one or more appropriate liposomes; (2) an estimate of a "distribution" parameter of a drug candidate may be determined from the binding interactions between the drug candidate and an appropriate set of plasma proteins (*e*.*g*., specific and nonspecific tissue binding and tissue permeability); (3) an estimate of a "metabolism" parameter of a drug candidate may be determined from the binding interactions between the drug candidate and an appropriate set of metabolic enzymes; and (4) an estimate of an "excretion" parameter of a drug candidate may be determined from the binding interactions between the drug candidate and an appropriate set of transport proteins.

Within the context of the present invention, suitable liposomes for estimating an "absorption" parameter of a drug candidate include organic compounds originating from natural or synthetic lipid molecules such as glycerophospholipids, glyceroglycolipids, sphingophospholipids and sphingoglycolipids, and from the classes phosphatidyl choline, phosphatidyl etanolamine, phosphatidyl serine, phosphatidyl glycerol, phosphatidyl acid, phosphatidyl inositol, galactopyranoside, digalactopyranoside, ceramide-phosphatidyl choline, ceramide-phosphatidyl etanolamine, ceramide-phosphatidyl serine, ceramide-phosphatidyl glycerol, ceramide-phosphatidyl acid, ceramide-phosphatidyl inositol, sphingomyelin molecules, glucosylceramides, glucocerebrosides, galactoceramides, galactocerebrosides, gangliosides, monoacyl phosphatidyl choline, cardiolipin molecules, that may be linked to saturated or unsaturated fatty or fluorocarbon chains ranging from eight to twenty-four carbons in length where fatty chains attached to the head group can be the same or of different structure, cholesterol, lanosterol, ergosterol, stigmasterol, sitosterol and derivatives thereof capable of being incorporated into lipid membranes, N,N-dimethyl-N-octadecyl-1-octadecanammonium chloride or bromide, (N-[1-(2,3-dioleoyloxy)propyl]-N,N,N-trimethylammonium chloride, diacetyl phosphate, N-[2,3-dihexadecyloxy)prop-1-yl]-N,N,N-trimethylammonium chloride, bolaamphiphiles, polyglycerolmonoalkylethers, polyethoxymonoalkylethers, as well as liposome-forming molecules from the classes amphiphilic polymers, amino acids, crown ether compounds and di(acyloxy)dialkylsilanes. The liposomes of the present invention and the like may be immobilized onto the sensing surface by the technique disclosed in Example 1.

Suitable plasma proteins for estimating a "distribution" parameter of a drug candidate include proteins such as Immunoglobulin G (7s-y-globulin), IgG; Immunoglobulin A, IgA; Secretory IgA, s IgA; Immunoglobulin M (19s-y-globulin) IgM; Immunoglobulin D, IgD; Immunoglobulin E, IgE; α1-Antitrypsin, α1 P1, α1 A; α1-Antichymotrypsin, (α1X-Glycoprotein) α1 X; Inter-α-Trypsin inhibitor, 1αTI; Antithrombin III, (heparin cofactor) AT III; α-Thiol Proteinase inhibitor (LMW kininogen) αTPI; C1-Inactivator, C1 esterase inhibitor (α2-neuraminoglycoprotein) C1 INA: α2-Macroglobulin, α2 M; α2-Antiplasmin, α2AP; Cystatin C (Post-y-globulin; y-Trace protein); C1q (11S protein); C1r; C1s; C2; C3 (β1C-globulin), C4 (β1E-globulin); C5 (β1F-globulin); C6; C7; C8; C9; Factor B, (C3-proactivator; β2-glycoprotein II; glycine-rich β-glycoprotein); Factor D (C3-proactivator convertase); Properdin, P; Factor I, (C3b inactivator); C4-binding Protein; Fibrinogen, FI, Prothrombin, F II; Factor V (proaccelerin); FV; Factor VII, (proconvertin), F VII; Factor VIII: C (antihemophilic factor) F VIII: C; Factor VIII - Related Antigen; FV III: Rag (von Willebrand factor) (VWF); Factor IX (Christmas factor) F IX; Factor X, (Stuart-Power factor) F X; Factor XI (plasma thromboplalstin antecedent) F XI; Factor XII (Hageman factor) F XII; Factor XIII (fibrin stabilizing factor) F XIII; high-molecular-weight (HMW) Kininogen (Fitzgerald factor); Prekallikrein (Fletcher factor); Plasminogen; Protein C; Protein S; Albumin, ALB; Haptoglobin, HP, Hp 1-1, Hp 2-1, Hp 2-2; Prealbumin (transthyretin, thyroxine-binding prealbumin); Retinol-binding Protein RBP; Thyroxine-binding Globulin TBG; Transcortin (corticosteroid-binding globulin) CBG; Sex hormone-binding globulin (Steroid-binding β Globulin) SHBG; Vitamin D-binding Protein (Gc-globulin, group specific component) VDBP; Transcobalamin I, TC I; Trancobalamin II, TC II; Transferrin (siderophilin) TF; Ferritin; Hemopexin, HPX; Apolipoprotein A, Apo A-1, Apo A-II; Apolipoprotein B, Apo B-48, Apo B-100; Apolipoprotein C; Apo C-I, Apo C-II, Apo C-III; Apolipoprotein E, Apo E; Apolipoprotein (a), apo (a); Serum Amyloid A, SAA; α-Fetoprotein, AFP; α1-Acid Glycoprotein (orosomucoid) α1 AG; Ceruloplasmin, CP; Serum Amyloid P protein (9.5S α1-glycoprotein; α1-macroglobulin) SAP; α2-HS Glycoprotein, α2 HS; Fibronectin (cold insoluble globulin) FN, C-reactive Protein, CRP; β2-microglobulin, β2 M; Pregnancy-specific β1-glycoprotein, SP1; α1-microglobulin, α1 M. The plasma proteins of the present invention and the like may be immobilized onto the sensing surface through the use of known immobilization techniques as is appreciated by those skilled in the art.

Suitable metabolic enzymes for estimating a "metabolism" parameter and/or an "excretion" parameter of a drug candidate include cytochrome P450-enzymes selected from the class of cytochrome P450 enzymes having an active site characterized by a protoporphyrin IX-iron complex with thiolate from a cysteine of the enzyme serving as the fifth ligand to iron. Suitable CYP 450 enzymes include CYTOCHROME P450, CYP1A1, CYP1A2, CYP2A1, 2A2, 2A3, 2A4, 2A5, 2A6, CYP2B1, 2B2, 2B3, 2B4, 2B5, 2B6, CYP2C1, 2C2, 2C3, 2C4, 2C5, 2C6, 2C7, 2C8, 2C9, 2C10, 2C11, 2C12, CYP2D1, 2D2, 2D3, 2D4, 2D5, 2D6, CYP2E1, CYP3A1, 3A2, 3A3, 3A4, 3A5, 3A7, CYP4A1, 4A2, 4A3, 4A4, CYP4A11, CYP P450 (TXAS), CYP P450 11A (P450scc), CYP P450 17(P45017a), CYP P450 19 (P450arom), CYP P450 51 (P45014a), CYP P450 105A1, CYP P450 105B1. Other important metabolic enzymes include Glutathione-thioethers, Leukotriene C4,butyrylcholinesterase, human serum paraoxonase/arylesterase, N-Acetyltransferase, UDP-glucuronosyltransferase (UDPGT) isoenzymes, TL PST, TS PST, drug glucosidation conjugation enzyme, the glutathione-S-transferases (GSTs) (RX:glutathione-R-transferase), GST1, GST2, GST3, GST4, GST5, GST6, alcohol dehydrogenase (ADH), ADH I, ADH II, ADH III, aldehyde dehydrogenase (ALDH), cytosolic (ALDH1), mitochondrial (ALDH2), monoamine oxidase, MAO: Ec 1.4.3.4, MAOA, MAOB, flavin-containing monoamine oxidase, enzyme superoxide dismutase (SOD), Catalase, amidases, N1,-monoglutathionyl spermidine, N1,N8-bis(glutathionyl) spermidine, Thioesters, GS-SG, GS-S-cysteine, GS-S-cysteinylglycine, GS-S-O3H, GS-S-CoA, GS-S-proteins, S-carbonic anhydrase III, S-actin, Mercaptides, GS-Cu(I), GS-Cu(II)-SG, GS-SeH, GS-Se-SG, GS-Zn-R, GS-Cr-R, Cholin esterase, lysosomal carboxypeptidase, Calpains, Retinol dehydrogenase, Retinyl reductase, acyl-CoA retinol acyltrunderase, folate hydrolases, protein phosphates (pp) 4 st, PP-1, PP-2A, PP-2Bpp-2C, deamidase, carboxyesterase, Endopeptidases, Enterokinase, Neutral endopeptidase E.C.3.4.24.11, Neutral endopeptidase, carboxypeptidases, dipeptidyl carboxypeptidase, also called peptidyl-dipeptidase A or angiotensin-converting enzyme (ACE) E.C.3.4.15.1, carboxypeptidase M, g-Glutamyl transpeptidase E.C.2.3.2.2, Carboxypeptidase P, Folate conjugase E.C.3.4.12.10, Dipeptidases, Glutathione dipeptidase, Membrane Gly-Leu peptidases, Zinc-stable Asp-leu dipeptidase, Enterocytic intracellular peptidases, Amino tripeptidase E.C.3.4.11.4, Amino dipeptidase E.C.3.4.13.2, Prodipeptidase, Arg-selective endoproteinase; the family of brush border hydrolases, Endopeptidase-24.11, Endopeptidase-2(meprin), Dipeptidyl peptidase IV, Membrane dipeptidase GPI, Glycosidases, Sucrase-isomaltase, Lactase-glycosyl-ceraminidase, Glucoamylase-maltase, Trehalase, Carbohydrase enzymes, alfa-Amylase (pancreatic), Disaccharidases (general), Lactase-phhlorizin hydrolase, Mammalian carbohydrases, Glucoamylase, Sucrase-Isomaltase, Lactase-glycosyl ceramidase, Enzymatic sources of ROM, Xanthine oxidase, NADPH oxidase, Amine oxidases, Aldehyde oxidase, Dihydroorotate dehydrogenase, Peroxidases, Human pancreatic exocrine enzymes, Trypsinogen 1, Trypsinogen 2, Trypsinogen 3, Chymotrypsinogen, proElastase 1, proElastase 2, Protcase E,Kallikreinogen, proCarboxypeptidase A1, proCarboxypeptidase A2, proCarboxypeptidase B1, proCarboxypeptidase B2, Glycosidase, Amylase, lipases, Triglycaride lipase, Collipase, Carboxyl ester hydrolase, Phospholipase A2, Nucleases, Dnase I, Ribonucleotide reductase (RNRs), Wistar rat exocrine pancreatic proteins, Label Protein IEP, A1 Amylase I, A2 Amylase 2, Lipase, CEL Carboxyl-ester lipase, PL Prophospholipase A, T1 Trypsinogen 1, T2 Trypsinogen 2, T3 Trypsinogen 3, T4 Trypsinogen 4, C1 Chymotrypsinogen 1, C2 Chymotrypsinogen 2, PE1 Proelastase 1, PE2 Proelastase 2, PCA Procarboxypeptidase A1, PCA1 Procarboxypeptidase A2, PCB 1 Procarboxypeptidase B1, PCB2 Procarboxypeptidase B2, R Ribonuclease, LS Lithostatin, Characteristics of UDPGT isoenzymes purified from rat liver, 4-nitrophenol UDPGT, 17b-Hydroxysteriod UDDPGT, 3-a-Hydroxysteroid UDPGT, Morphine UDPGT, Billirubin UDPGT, Billirubin monoglucuronide, Phenol UDPGT, 5-Hydroxytryptamine UDPGT, Digitoxigenin monodigitoxide UDPGT, 4-Hydroxybiphenyl UDPGT, Oestrone UDPGT, Peptidases, Aminopeptidase N, Aminopeptidase A, Aminopeptidase P, Dipeptidyl peptidase IV, b-Casomorphin, Angiotensin-converting enzyme, Carboxypeptidase P Angiotensin II, Endopeptidase-24.11, Endopeptidase-24.18 Angiotensin I, Substance P (deamidated), Exopeptidase,1. NH2 terminus Aminopeptidase N (EC 3.4.11.2), Aminopeptidase A (EC 3.4.11.7), Aminopeptidase P (EC 3.4.11.9), Aminopeptidase W (EC 3.4.11.-), Dipeptidyl peptidase IV (EC 3.4.14.5), g-Glutamyl transpeptidase (EC 2.3.2.2), 2. COOH terminus Anglotensin-converting enzyme (EC 3.4.15.1), Carboxypeptidase P (EC 3.4.17.-), Carboxypeptidase M (EC 3.4.17.12),3. Dipeptidase Microsomal dipeptidase (EC 3.4.13.19), Gly-Leu peptidase, Zinc stable peptidase,Endopeptidase Endopeptidase-24.11 (EC 3.4.24.11), Endopeptidase-2 (EC 3.4.24.18, PABA-peptide hydrolase, Meprin, Endopeptidase-3, Endopeptidase (EC 3.4.21.9), GST A1-1, Alpha,GST A2-2 Alpha, GST M1a-1a Mu, GST M1b-1b Mu, GST M2-2 Mu, GST M3-3 Mu, GST M4-4 Mu, GST M5-5 Mu, GST P1-1 Pi, GST T1-1 Theta, GST T2-2 Theta, Microsomal Leukotriene C4 synthase, UGT isozymes, UGT1.1, UGT1.6, UGT1.7, UGT2.4, UGT2.7, UGT2.11, Pancreatic enzymes, Elastase, Aminopeptidase (dipeptidyl aminopeptidase (IV), Chymotrypsin, Trypsin, Carboxypeptidase A, Methyltransferases, O-methyltransferases, N-methyltransferases, S-methyltransferases, Catechol-O-methyltransferases, MN-methyltransferase, S-sulphotransferases, Mg²⁺-ATPase, Growth factor receptors Alkaline phosphatase, ATPases, Na, K+ATPase, Ca²⁺-ATPase, Leucine aminopeptidase, K⁺channel. The metabolic enzymes of the present invention and the like may be immobilized onto the sensing surface through the use of known immobilization techniques as is appreciated by those skilled in the art.

Suitable transport proteins for estimating an "excretion" (as well as an efflux, absorption, distribution) parameter of a drug candidate include Glucose a. Na⁺-glucose cotransp GLUT 1, b. Facilitative transporter, GLUT 1-5, GLUT-2, Neutral amino acid transporter, Na⁺-independent system L amino acid transporter, cationic amino acid, Y⁺ cationic L-amino acid transporter, Dipeptides H⁺ contransport, Nucleosides Na⁺-dependent and facilitative, Taurine Na⁺ and Cl⁻ dependent, Bile acids Na⁺/bile acid cotransporter, Na⁺-independent bile acid transporter, ABC transporters, Prostaglandins facilitative transpoprter, Na⁺/H⁺ exchanger Antiporter, Phosphate Na⁺/Pi cotransporter, Sulfate Na⁺-cotransporter, Transporters for neurotransmitters, Norepinephrine Na⁺/Cl⁻ cotransporter, Dopamine Na⁺/Cl⁻ cotransporter, Serotonine Na⁺/Cl⁻ cotransporter, GABA, GAT-1, Na⁺/Cl⁻ dependent, Glycine Na⁺/Cl⁻ dependent, Glutamate Na⁺ cotransporter, K⁺/OH⁻ counter-transport, ABC transporters, P-glycoprotein (MDR1, MDR 2 OR MDR 3), Cl- channel (CFTR), Antigenic peptides, TAP1 and TAP2 heterodimer, Lung resistance protein (LRP), Multidrug resistance protein 1 (MRP1), Multidrug resistance protein 2 (MRP2 or cMOAT), Multidrug resistance protein 3 (MRP3), Multidrug resistance protein 4 (MRP4), Multidrug resistance protein 5 (MRP5), Multidrug resistance protein 6 (MRP6), mrp (mouse), EBCR (rabbit), C. elegans mrp1 (nematode), C. elegans mrp2 (nematode), MRP6 (human), YCF (yeast), AtMRP (Arabidopsis), SUR1 (human), sur2 (rat, mouse), YOR1/YRS1 (yeast), LtpgpA (leishmania), Hepatic amino acid transport system, Neutral amino acid transporters, MeAIB, Dicarboxylic amino acids, Neutral amino acids (branched), b-amino acids, Long chain fatty acids, Monoglycerides, L-Lysophosphatidylcholine, Transport proteins for bilirubin, Bilitranslocase (BTL), Organic anion binding protein (OABP), BSP/bilirubin binding protein, Signal receptor and transduction-hydrolases, ATPases, Na⁺dependent/independent bile acid transport, Bilirubin/BSP carrier (Cl-dependent), SO/OH exchanger Cl⁻channel Na⁺/H⁺ exchanger, Na⁺/HCO³ cotransport, GSH-, GSSH-, GS conjugate carrier, SO⁴/HCO³ exchanger, Na⁺-dependent amino acid transport, Dipolar amino acid transporter, Basic amino acids, Cystine, Imino acids Cl⁻, b-Amino acids Cl⁻, XAG Acidic amino acids K⁺, A Dipolar a-amino acids, three-and four-carbon dipolar amino acids, L Bulky, hydrophobic, dipolar amino acids, y+ Basic amino acids, folate transperter, cbl transport proteins, Na⁺-K⁻ - ATPase, Bile acid transporter (BAT), protein kinase C, Na⁺/I⁻ sympoter (NIS); Bile salt export pump(BSEP, cBAT, SPGP); Intestinal bile acid transporters; Purine selective Na⁺-dependent nucleoside transporter (hSPNTI); Pyrimidine selective Na⁺-dependent nucleoside transporter (cNTI); Mitoxantrone transporter (MXR1 and MXR2); Intestinal oligopeptide transporter(PepT1); Renal oligopeptide transporter(PepT2); Breast cancer resistance protein (BCRP). The transport/efflux proteins of the present invention and the like may be immobilized onto the sensing surface through the use of known immobilization techniques as is appreciated by those skilled in the art.

As stated above, it has been discovered that the pharmacokinetic parameters of absorption, distribution, metabolism, and excretion (ADME) of a drug candidate may be determined from the binding interactions between the drug candidate and appropriate sensing surface-bound biomolecules (*e*.*g*., from different liposomes, plasma proteins, CYP 450 enzymes, other metabolic enzymes, and/or transport/efflux proteins, such as those as identified above) of a biosensor. That is, the binding interactions between a drug candidate and one or more sensing surface-bound biomolecules selected from liposomes, plasma proteins, CYP 450 enzymes, other metabolic enzymes, and transport/efflux proteins, may be measured via the BIACORE instrument to determine a "binding interaction parameter" of the drug candidate. The estimated binding interaction parameter may then, in turn, be compared against a predetermined drug correlation graph to estimate one or more absorption, distribution, metabolism, and excretion "pharmacokinetic parameters" of the drug candidate. Moreover, the pharmacokinetic parameter of the drug candidate may be, for example, selected from the group consisting of volume of distribution; total clearance; protein binding; tissue binding; metabolic clearance; renal clearance; hepatic clearance; biliary clearance; intestinal absorption; bioavailability; relative bioavailability; intrinsic clearance; mean residence time; maximum rate of metabolism; Michaelis-Menten constant; partitioning coefficients between tissues and blood (or plasma) such as those partitioning coefficients associated with the blood brain barrier, blood placenta barrier, blood human milk partitioning, blood adipose tissue partitioning, and blood muscle partitioning; fraction excreted unchanged in urine; fraction of drug systemically converted to metabolites; elimination rate constant; half-life; and secretion clearance; any one of which may be determined from appropriate sensorgrams of the selected drug-biomolecule interaction (alternatively, they may be determined by extracting score vectors with principal component analysis from digitalized interaction profiles, as well as other known multivariate methods).

For example, to estimate an apparent equilibrium constant between a drug candidate and selected sensing surface-bound biomolecules, the following procedure may be employed. First, a concentration series (*e*.*g*., 0, 20, 50, 100, 500, and 1,000 µM) of the drug candidate may be prepared, and sequentially injected into a biosensor having a sensor chip operatively associated therewith, wherein the sensor chip has a reference sensing surface and at least one sensing surface with surface-bound biomolecules. The relative responses at steady-state binding levels for each drug concentration level may then be measured. Because of bulk-refractive index contributions from solvent additives in the biosensor's running buffer, a correction factor may be calculated (via known calibration procedures) and applied to give corrected relative responses. The corrected relative responses for each drug concentration may then be mathematically evaluated as is appreciated by those skilled in the art to estimate the apparent equilibrium constant.

More specifically, the apparent equilibrium constant (KD) between the drug candidate and the selected sensing surface-bound biomolecules may be calculated by fitting the measured equilibrium (R_{eq}) data and known drug concentration (C) data to Equation (1):${\text{R}}_{\text{eq}} {\text{= C*R}}_{\text{max}} \text{/(C + KD) + offset}$ wherein Rₘₐₓ is the maximum binding capacity of the sensing surface. Alternatively, the apparent equilibrium constant (KD) may be calculated from as little as two concentrations (*i*.*e*., C1 and C2) of the drug candidate by use of Equation (2):${\text{KD = (R}}_{\text{eqC1}} {\text{/C1 - R}}_{\text{eqC2}} {\text{/C2)/(R}}_{\text{eqC2}} {\text{- R}}_{\text{eqC1}} \text{)}$

The estimated apparent equilibrium constant (KD) or binding level at a specific molar drug concentration may then, in turn, be compared against a predetermined drug correlation graph to estimate one or more absorption, distribution, metabolism, and excretion parameters of the drug candidate.

In the context of the present invention, a predetermined drug correlation graph refers to a mathematical expression or function that has been developed from binding interaction data associated with known drug compounds. For example, a correlation graph may be constructed wherein known binding interaction parameters (*e*.*g*., apparent equilibrium constants and/or binding levels at specific molar drug concentrations) for known compounds are plotted along the abscissa (*i*.*e*., the x-axis) and corresponding measured binding interaction parameters obtained via the biosensor are plotted along the ordinate (*i*.*e*., the y-axis), or vice versa. Stated somewhat differently, the correlation plot is a graphical representation of a mathematical expression that correlates known and measured affinity data.

By comparing the estimated binding interaction parameter obtained from the selected drug-biomolecule interaction against the mathematical expression (*i*.*e*., correlation graph) correlated from known and measured affinity data, it has been surprisingly discovered that an estimate of a pharmacokinetic ADME parameter related to the drug candidate may be accurately predicted. In addition, by immobilizing a selected combination of at least two different liposomes, plasma proteins, CYP 450 enzymes, other metabolic enzymes, and/or transport/efflux proteins, onto one or more discrete sensing surfaces of a sensor chip, it has been further surprisingly discovered that at least two pharmacokinetic parameters associated with a drug candidate may be readily determined. Moreover, by immobilizing a selected combination of different liposomes, plasma proteins, CYP 450 enzymes, other metabolic enzymes, and/or transport/efflux proteins, onto the sensing surfaces of biosensor, such as in a predetermined line of different spots of biomolecules, an ADME pattern or profile, such as a drug candidate characterization matrix, may be readily developed. Such ADME profiles are of great utility for purposes of drug screening.

In addition to determining one or more pharmacokinetic parameters by monitoring the refractive index changes of a biosensor as disclosed above, the solubility of a drug candidate may also be simultaneously determined (together with the one or more pharmacokinetic parameters) by monitoring the minimum, maximum or centroid of the drug-biomolecule interaction signal (as is disclosed, for example, in PCT Publication No. WO 97/09618). More specifically, the solubility of the drug candidate may be estimated/identified from irregularities, as well as from reflectance minimum (Rₘᵢₙ) and dip-shape data, shown in the sensorgram of the drug-biomolecule interaction. In general, the concentration at which precipitation occurs is referred to as the solubility limit; this property may be important to measure because it may indicate that the drug candidate has an affinity greater than otherwise indicated.

Solubility problems of the drug candidate may be detected because insoluble particles (*i*.*e*., precipitates) tend to bind to sensing surface-bound biomolecules, thereby causing the sensing surface to be non-homogenous. (A homogenous surface has the same surface concentration throughout, whereas a non-homogenous surface has concentration disruptions.) A non-homogenous sensing surface actually measures several refractive indices, which are all averaged together in the biosensor's detector. These multiple measurements from a single, but non-homogenous sensing surface, tend to result in an increase in reflectance minimum and a broadening of the dip associated therewith.

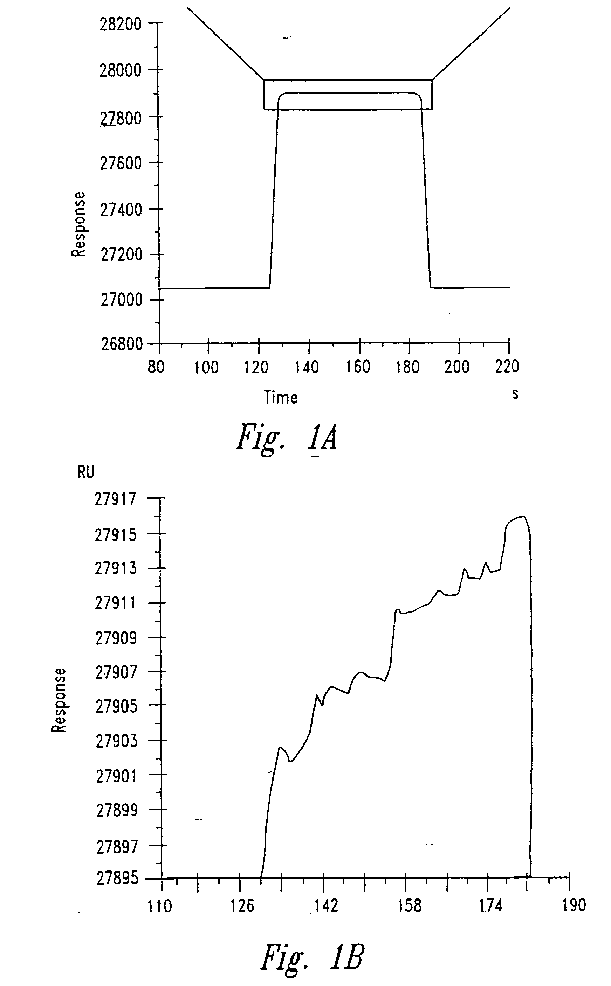

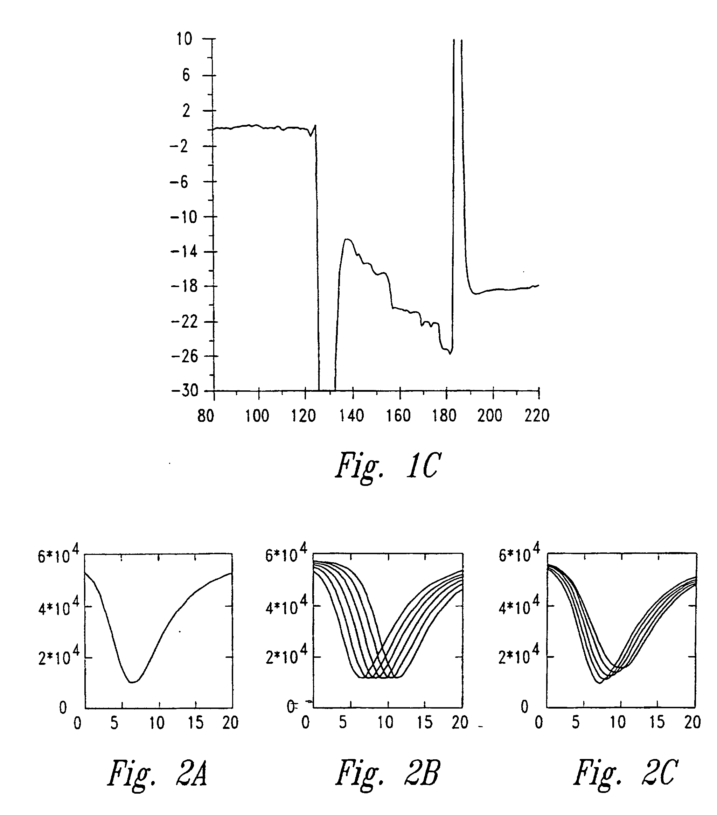

An illustration of how sensorgram irregularities may be used to identify solubility problems (*i*.*e*., the presence of precipitates) is shown in Figures 1A-C. More specifically, the steady-state binding levels associated with a selected drug-biomolecule interaction is generally shown in Figure 1A. By enlarging or "zooming" in on the top area of the sensorgram, which is reflective of the steady-state binding, the sensorgram irregularities become more apparent as is shown in corresponding Figure 1B. The sensorgram irregularities become even more apparent by using reference subtracted data (*i*.*e*., bulk-refractive index effects have been eliminated) as is shown in corresponding Figure 1C.

An illustration of how reflectance minimum (Rₘᵢₙ) and dip-shape data may be used to identify solubility problems (*i*.*e*., the presence of precipitates) is shown in Figures 2A-C. More specifically, a homogeneous surface has the same surface concentration throughout, and will thus result in a single "dip" with respect to the reflected light intensity as shown in Figure 2A. When precipitates bind to the sensing surface, the sensing surface becomes non-homogenous which tends to result in an increase in reflectance minimum and a broadening of the dip associated therewith as is shown in Figures 2B and 2C, respectively.

Based on the foregoing methods for assaying a drug candidate, researchers may now simultaneously measure several different pharmacokinetic parameters of the drug candidate, as well as gauge the drug candidate's solubility, by using a single analytical instrument. The present invention simplifies and improves the rate of drug discovery and development because important pharmacokinetic data may now be readily obtained at a relatively early stage of the process.

In addition to the foregoing methods, the present invention is also directed to apparatuses adapted to carrying out such methods. More specifically, the apparatuses of the present invention comprise a biosensor having a sensing surface associated therewith, and a computer system that facilitates the implementation of the steps associated with the methods disclosed herein. The computer includes a computer memory containing a data structure useful for assaying a drug candidate; the data structure comprises binding interaction data associated with known drug compounds such that the data structure may be used to determine an estimate of at least pharmacokinetic parameter of the drug candidate.

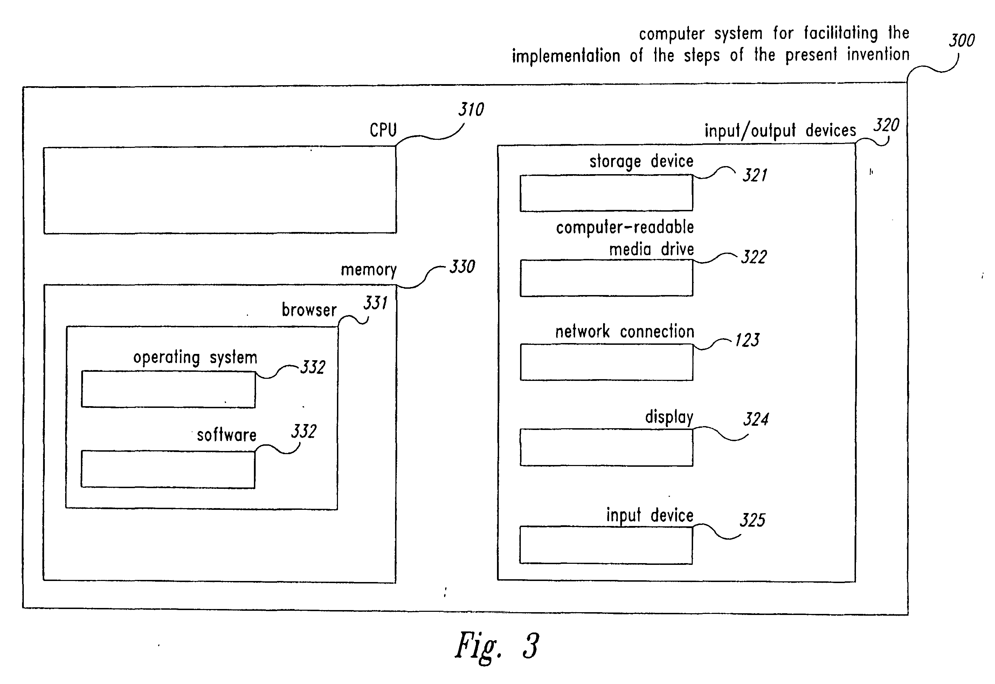

The computer memory containing a data structure useful for assaying a drug candidate may be more fully illustrated in the context of a high-level computer block diagram as is depicted in Figure 3.

As shown, such a computer system 300 contains one or more central processing units (CPUs) 310, input/output devices 320, and the computer memory containing a data structure useful for assaying a drug candidate(memory) 330. Among the input/output devices is a storage device 321, such as a hard disk drive, and a computer-readable media drive 322, which may be used to install software products, where the software products are provided on a computer-readable medium, such as a CD-ROM. The input/output devices also include a network connection 323, through which the computer system 300 may communicate with other connected computer systems, such as networks. The input/output devices may also contain a display 324 and a data input device 325.

The memory 330 preferably contains an operating system 331, such as MICROSOFT WINDOWS, for providing to other programs access to resources of the computer system. The memory 330 preferably further contains software 332. While the computer memory containing a data structure useful for assaying a drug candidate is preferably implemented on a computer system configured as described above, those skilled in the art will recognize that it may also be implemented on computer systems having different configurations.

The present invention may use a generated data signal conveying a data structure useful for assaying a drug candidate. As above, the data structure comprises binding interaction data associated with known drug compounds, such that the data structure may be used to determine an estimate of at least pharmacokinetic parameter of the drug candidate.

A sensor surface adapted for use with a biosensor has a hydrogel matrix coating coupled to the top surface of the sensor surface, wherein the hydrogel matrix coating has a plurality of functional groups, and at least two different types of liposomes are bonded to the plurality of functional groups at discrete and noncontiguous locations on the hydrogel matrix coating of the sensor surface (such as disclosed in Example 1). In the context of the BIACORE instrument as described above, the sensor surface is preferably in the form a sensor chip, wherein the sensor chip has a free electron metal interposed between the hydrogel matrix and the top surface of the sensor chip. Suitable free electron metals in this regard include copper, silver, aluminum and gold.

The sensor surface may have a lipophilic substance interposed between the different types of liposomes and the plurality of functional groups, wherein the lipophilic substance is covalently bonded to the plurality of functional groups. Representative lipophilic substances comprise an alkyl chain having from 12 to 24 carbon atoms, such as stearylamine. Similarly, representative liposomes include 1,2-dimyristol-*sn*-glycero-3-phosphocholine (DMPC) and 1-palmitoyl-2-oleoyl-*sn*-glycero-3-phosphocholine (POPC). The sensor surfaces of this invention may also have non-liposome biomolecules associated therewith. For example, human serum albumin, CYP 450 enzyme, a metabolic enzyme, and/or a transport protein may be bonded to the plurality of may be bonded to the plurality of functional groups at discrete and noncontiguous locations on the hydrogel matrix coating of the sensor surface.

For purposes of illustration and not limitation, the following examples more specifically disclose various aspects of the present invention.

### EXAMPLES

### EXAMPLE 1

### SIMULTANEOUS MEASUREMENT OF SOLUBILITY, PLASMA PROTEIN BINDING, LIPOPHILICITY AND INTESTINAL ABSORPTION FOR THREE DRUG CANDIDATES A-C

This example discloses how combined information from each of four different flow-cells of a biosensor may be represented in a characterization matrix where each of the three drug candidates A-C illustrates a quality pattern (*i.e*., HSA% Bound, Predicted Lipophilicity, Solubility, and Predicted FA%) which is useful for the selection of lead drug compounds.

### Preparation of Sensor Chip

Three of the four discrete sensing surfaces of a CM5 Sensor Chip (Biacore AB, Uppsala, Sweden) were modified such that the CM5 Sensor Chip had surface-bound biomolecules as depicted below in Table 1.

**TABLE 1**

| SURFACE-BOUND BIOMOLECULES OF CM5 SENSOR CHIP | | |
|---|---|---|
| Surface/Cell No. | Type of Flow-Cell | Surface Modification |
| FC 1 | Ref-1 | Unmodified carboxymethyl dextran (CM5) |
| FC2 | Target-1 | Human Serum albumin - HSA (9-12 kRU) |
| FC3 | Target-2 | DMPC-liposomes (5-7 kRU) captured on stearylamine |
| FC4 | Target-3 | POPC-liposomes (5-7 kRU) captured on stearylamine |

More specifically, and to achieve covalent attachment of stearylamine to surface/cell nos. 3 and 4, the following procedure was employed. A CM5 Sensor Chip was first inserted into a BIACORE 3000 biosensor (Biacore AB, Uppsala, Sweden). The flow of running buffer (isotonic phosphate buffer pH 7.0 (9.6 g Na₂HPO₄ - 2H₂O, 1.7 g KH₂PO₄, 4.1 g NaCl to 1 liter)) with 2% dimethylsulfoxide (DMSO) was directed to flow-cells 3 and 4 by using appropriate software commands. A mixture of 0.2 M N-ethyl-N'-(3-diethylaminopropyl)-carbodiimide and 50 mM N-hydroxysuccinimide in water was then injected into the biosensor so as to flow over flow-cells 3 and 4 for a period of 10 minutes. The CM5 Sensor Chip was then washed with running buffer, removed from the biosensor, and placed in a petri dish on top of a few layers of tissue paper that had been moistened with ethanol. The exposed sensing surfaces were then treated with 30 µl of a 10 mM stearylamine solution in 99% ethanol. After another 45 minute period, the CM5 sensor chip was gently washed with ethanol and then with water. Next, the exposed sensing surfaces were further treated with 50 µl of a 1M ethanolamine solution at pH 8.5. Finally, and after another 10 minute period, the sensing surfaces were washed with HBS-buffer.

Following covalent attachment of stearylamine to surface/cell nos. 3 and 4 - and after the CM5 Sensor Chip that had just been washed with HBS-buffer was blown dry with nitrogen and reinserted into the biosensor - human serum albumin (HSA), DMPC-liposomes, and POPC-liposomes (available from Sigma) were then captured onto surface/cell nos. 2, 3 and 4, respectively. The following procedures were employed to capture these biomolecules.

To capture human serum albumin the flow was initially directed to flow-cell 2 by using appropriate software commands. The sensing surface of flow-cell 2 was then activated for a period of 7 minutes with EDC/NHS. Next, human serum albumin was injected at 15 µg/ml in 10mM acetate buffer pH 5.2 for a period of 7 minutes; then 1M ethanolamine pH 8.5 was injected for another period of 7 minutes. This procedure resulted in the immobilization of 9-12 kRU of human serum albumin to the sensing surface of surface/cell no. 2.

To capture the two different liposomes the flow was first directed to flow-cell 3, and 0.5-1 mM DMPC liposome was injected until 5-7 kRU of the DMPC liposome had been captured. The flow was then directed to flow-cell 4, and 0.5-1 mM POPC liposome was injected until 5-7 kRU of the POPC liposome had been captured. Finally, the flow-system of the biosensor was washed with 100mM NaOH, and the autoinjector tubing was washed with 0.5% SDS and 50 nM glycine pH 9.5 to remove trace lipids.

### Calibration Procedure

In order to improve data quality by reducing bulk-refractive index contributions from the DMSO solvent additive, a calibration procedure was employed. (Note the calibration procedure required varying concentrations of DMSO.) Because the running buffer contained 2% DMSO, a series of 8 to 10 calibration solutions were made that had varying DMSO concentrations ranging from 1.5% to 3.0%. Each calibration solution was sequentially injected over each of the four sensing surfaces by using the serial injection mode of the biosensor, and the respective steady-state binding levels were measured. The relative responses of the reference sensing surface of flow-cell 1 (FC1 having unmodified CM5 as a reference surface) were then subtracted from the corresponding calibration responses of the target sensing surfaces of flow-cells 2, 3, and 4, respectively, and plotted as functions of the relative responses. From these plots, calibration functions were calculated for each of the respective sensing surfaces.

The calibration functions were then used to calculate appropriate correction factors for the samples containing the three drug candidates A-C. That is, for the samples containing the drug candidates, the relative responses of the steady state binding levels of the reference sensing surface (*i*.*e*., signals from flow-cell 1) were measured and the respective calibration functions were used to calculate correction factors appropriate for each drug candidate. The correction factors were then applied (*i*.*e*., subtracted) to the differences between the reference responses and the sample responses to give a responses where the bulk-refractive index contributions from the DMSO solvent additive had been eliminated.

### Corrected Relative Responses of Drug Candidates A-C

The modified CM5 Sensor Chip (having surface-bound biomolecules as depicted above in Table 1) was used to measure the binding interaction of the three drug candidates A-C. Concentration series (*i*.*e*., 0, 20, 50, 100, 500, and 1,000, µM) of each drug candidate were prepared, and sequentially injected in serial mode into the biosensor such that four sensorgrams corresponding to each flow-cell were developed. Corrected relative responses at steady-state binding levels for each drug concentration were determined for each of the target sensing surfaces/flow-cells; such data is presented below in Tables 2-4.

**TABLE 2**

| DRUG CONCENTRATION DATA FOR HUMAN SERUM ALBUMIN | | | | | |
|---|---|---|---|---|---|
| Candidate | 10 µM | 50 µM | 100 µM | 500 µM | 1,000 µM |
| A | 50 | 110 | 130 | 140 | 145 |
| B | 0 | 0 | 0 | 10 | 50 |
| C | 10 | 50 | 100 | 110 | 115 |

**TABLE 3**

| DRUG CONCENTRATION DATA FOR POPC LIPOSOME | | | | | |
|---|---|---|---|---|---|
| Candidate | 10 µM | 50 µM | 100 µM | 500 µM | 1,000 µM |
| A | 10 | 300 | 400 | 450 | 500 |
| B | 0 | 0 | 40 | 60 | 70 |
| C | 0 | 0 | 0 | 0 | 10 |

**TABLE 4**

| DRUG CONCENTRATION DATA FOR DMPC LIPOSOME | | | | | |
|---|---|---|---|---|---|
| Candidate | 10 µM | 50 µM | 100 µM | 500 µM | 1,000 µM |
| A | 0 | 50 | 70 | 90 | 95 |
| B | 0 | 10 | 20 | 50 | 55 |
| C | 0 | 0 | 0 | 0 | 0 |

### Analysis of Data

The corrected relative responses for each drug concentration as depicted above in Tables 2-4 were, among other things, adjusted for differences in molecular weight of the drug candidates, as well as transformed to increase correlation with other properties. For example, for each target sensing surface/flow-cell, an apparent equilibrium constant (KD) was calculated by fitting the measured equilibrium (R_{eq}) data and known drug concentration (C) data to Equation (3):${\text{R}}_{\text{eq}} {\text{= C*R}}_{\text{max}} \text{/(C + KD) + offset}$ wherein Rₘₐₓ is the maximum binding capacity of the sensing surface. In the fitting procedure, Rₘₐₓ, KD, and the offset were calculated.

Alternatively, the apparent equilibrium constant (KD) may have been calculated from two concentrations of the drug candidate, C1 and C2, by use of Equation (4):${\text{KD = (R}}_{\text{eqC1}} {\text{/C1 - R}}_{\text{eqC2}} {\text{/C2)/(R}}_{\text{eqC2}} {\text{- R}}_{\text{eqC1}} \text{)}$

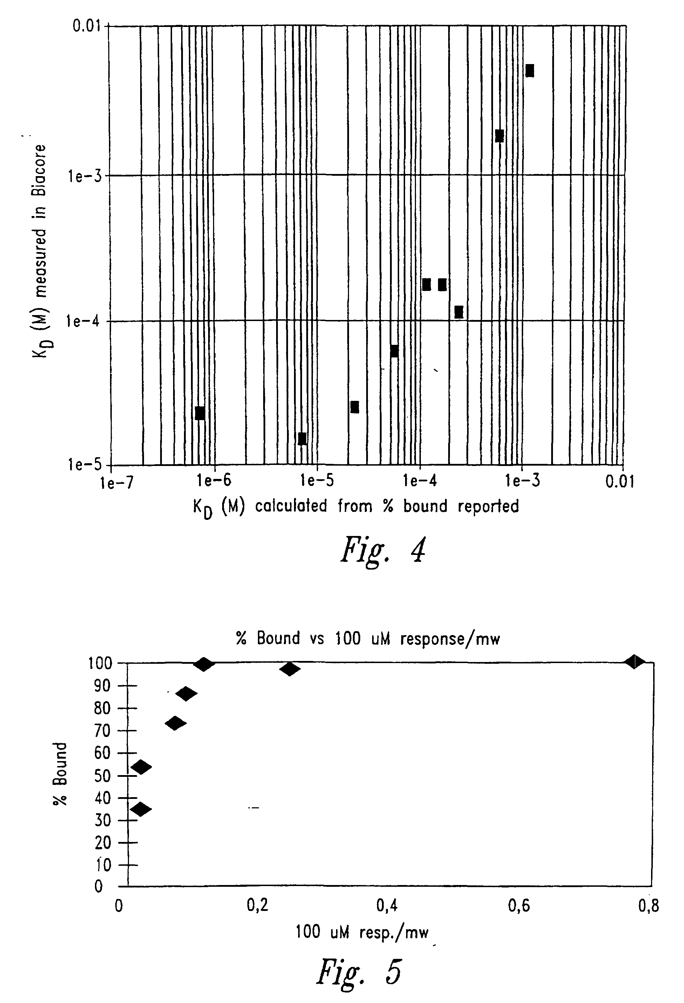

In either case, the KD values obtained via the biosensor were subsequently correlated with known KD values calculated from reported human serum albumin binding percentages (*i*.*e*., reported binding percentages that have been reported for known drug compounds), thereby enabling a prediction of the degree of protein binding for each of the three drug candidates A-C. In other words, a correlation graph as shown in Figure 4 was constructed having known KD values for known compounds plotted along the abscissa (*i*.*e*., the x-axis) and corresponding measured KD values obtained via the biosensor plotted along the ordinate (*i*.*e*., the y-axis). This correlation graph was then used to predict the degree of plasma protein binding for each of the three drug candidates A-C (see below).

In addition, for each target sensing surface/flow-cell, a molecular weight adjusted response at a single concentration level was used as a threshold value for ranking the drug candidates A-C. More specifically, a correlation graph as shown in Figure 5 was constructed, wherein respective binding levels at 100 µM (R 100 µM) divided by the known drug compounds' molecular weight were plotted along the abscissa (*i.e*., the x-axis) and corresponding human serum albumin binding percentage, as measured by equilibrium dialysis, were plotted along the ordinate (*i*.*e*., the y-axis). This correlation graph was then used to discriminate between strong and weak human serum albumin binders for each of the three drug candidates A-C.

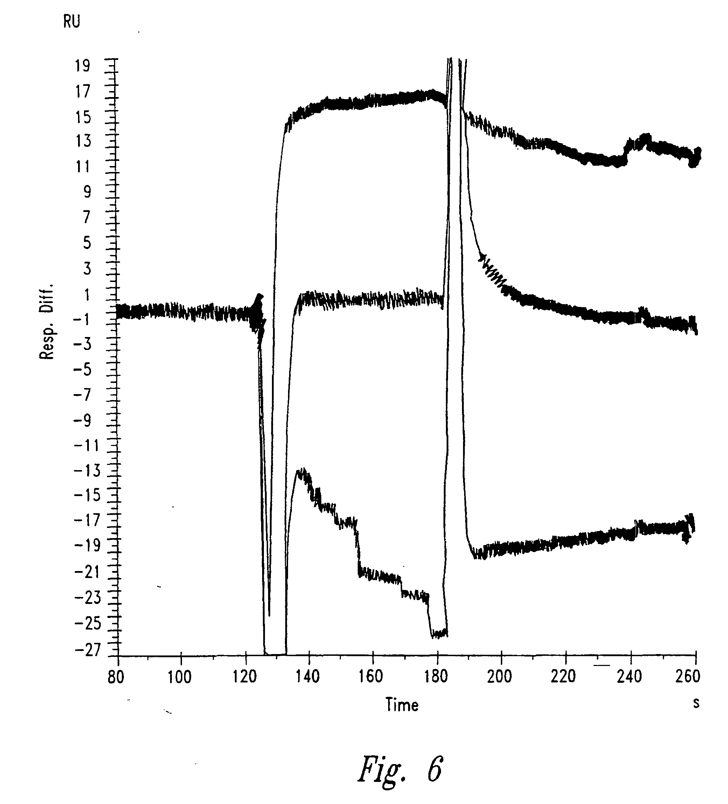

Furthermore, for each target sensing surface/flow-cell, reference subtracted sensorgram traces (*e*.*g*., FC2 minus FC1) were developed to detect the presence of precipitates (caused by low solubility). More specifically, reference subtracted sensorgram traces for each of the three drug candidates A-C, as shown in Figure 6, were constructed. (Note that when precipitates form and then bind/associate with the sensing surface, the response signal will decrease in a non-continuous way.) By analyzing the concentration series for each of the three drug candidates A-C (as shown in Figure 6), it was determined that drug candidate A precipitated and thus had relatively low solubility, whereas drug candidates B-C did not precipitate and thus had relatively high solubilities.

Finally, and as shown below in Table 5, the combined information from each target sensing surface/flow cell was tabulated into a reduced characterization matrix where each drug candidate A-C received a quality pattern that was useful for selection of the lead drug compounds. (Note that the predicted lipophilicity and fraction absorbed are prophetic.)

**TABLE 5**

| CHARACTERIZATION MATRIX FOR DRUG CANDIDATES A-C | | | | |
|---|---|---|---|---|
| Candidate | HSA % Bound | Pred. Lipophilicity | Solubility | Predicted FA% |
| A | 99.9 | 4 | <1 µM | >97 |
| B | <90 | 2 | OK | >90 |
| C | >90 | -2 | OK | <50 |

Based on the forgoing characterization matrix, it was determined that drug candidate B was the preferred compound due to its low plasma protein binding level (HSA %bound <90), medium lipophilicity (predicted lipophilicity = 2), no identified solubility problems (solubility = OK), and acceptable intestinal absorption (predicted fraction absorbed >90).

### EXAMPLE 2

### DEMONSTRATED CORRELATION BETWEEN BIOSENSOR DATA AND FRACTION ABSORBED IN HUMANS (FA%)

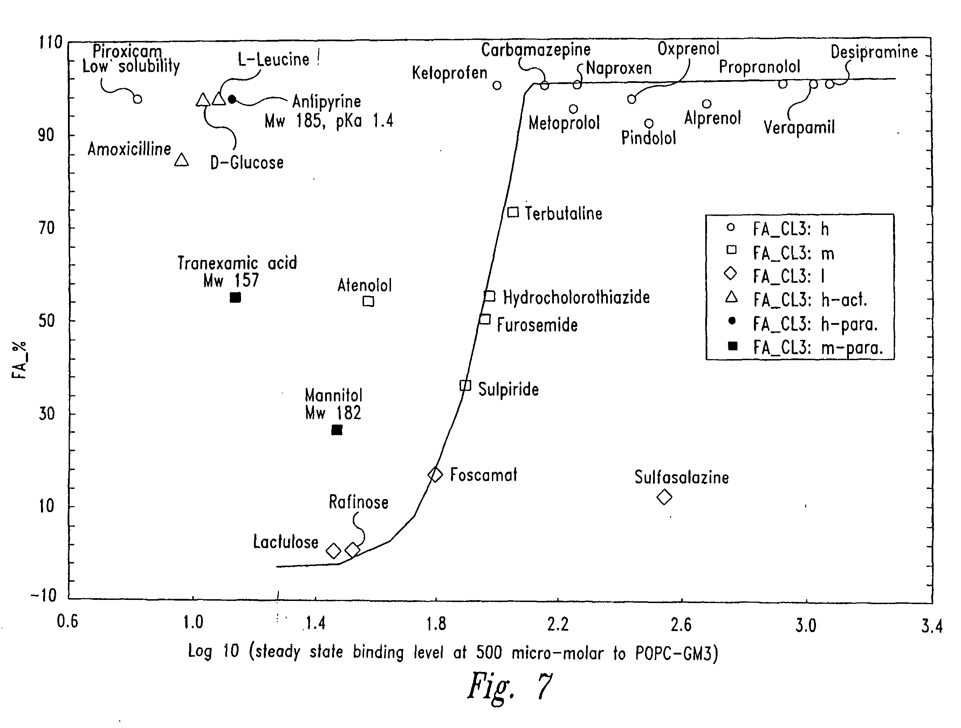

This example discloses a correlation between biosensor data obtained form the BIACORE instrument (*i*.*e*., BIACORE 3000) and known data for the fraction absorbed in humans (FA%) for a number of different drugs, wherein the correlation graph is useful for drug candidate absorption predictions.-More specifically, a correlation graph as shown in Figure 7 was constructed having known fraction absorbed in humans (FA%) plotted along the ordinate (*i*.*e*., the y-axis) and corresponding calibrated (*i*.*e*., reference subtracted) steady state binding levels for each drug at 500 µM plotted along the abscissa in a 10-logarithm scale (*i*.*e*., the x-axis). In this example, the sensing surfaces of the target flow-cells each had 6,000 RU of POPC-GM3 ganglioside (available from Sigma) captured on stearylamine tiles. (Note that an unmodified sensing surface of a CM5 Sensor Chip was used as the reference.) As shown in Figure 7, there is high degree of correlation between the biosensor data and the known fraction absorbed in humans (FA%) data, as is evidenced by the mathematical expression/function that has been fitted to the various data points.

Moreover, the correlation graph also shows a classification of the various substances, wherein absorption of the substances using the passively transported trans-cellular route through the intestine are depicted by h = high, m = medium, and 1 = low, and wherein absorption using active transport is depicted by h-act = high, and wherein absorption of substances with molecular weights <200 using the para-cellular route is depicted by h-para = high and m-para = medium, respectively. (Note the Sulfasalazine is a pre-drug which rapidly decomposes in the intestine, which may explain its outlier properties; Piroxicam has a very low solubility <<<<500 µM, which may explain its lack of correlation.)

### EXAMPLE 3

### BIOSENSOR ANALYSIS OF THE INTERACTION BETWEEN IMMOBILIZED HUMAN SERUM ALBUMIN AND DRUG COMPOUNDS FOR PREDICTION OF HSA

Many compounds bind reversibly to human serum albumin (HSA), α₁-acid glycoprotein (AGP) and other serum components. The affinity of a drug toward plasma proteins is an important issue when determining its overall pharmacokinetic profile. A high level of protein binding reduces the free concentration and therefore the physiological activity of the drug. Circulating protein-drug complex also serve to replenish the free drug concentration as free drug is removed from the body by various elimination processes, and thereby prolong the duration of drug action. Thus, the level of protein binding is an important factor in the delicate balance between intended physiological activity and potential side effects of the drug. Ideally the free concentration of each drug candidate should be estimated in the blood from the patient for which the drug is intended. Early estimates of the protein binding level, as well as of other ADME parameters, are now becoming more important in the drug discovery process. Therefore, simplified approaches for the determination of protein binding are being considered. A wide variety of methods and experimental conditions are used for this purpose. Methods include equilibrium dialysis, ultrafiltration, ultracentrifugation, spectroscopic methods, affinity and size exclusion chromatography and electrophoretic methods as reviewed by Oravcová (Oravcová et al., "Drug-Protein Binding Studies. New Trends in Analytical and Experimental Methodology," *J. Chromatogr. 677:*1-28 (1996)) and Hage (Hage et al., "Review. Recent Advances in Chromatographic and Electrophoretic Methods for the Study of Drug-Protein Interactions," *J. Chromatogr., 699*:499-525 (1997)). These methods rely either on a separation of bound and free drug or protein, or on a change in intrinsic parameters such as the mobility of protein or the spectroscopic properties of a drug-protein complex. To simplify the studies of drug protein interactions it is also common to use single proteins, HSA and/or α₁-acid glycoprotein, instead of blood or plasma. This approach is particularly useful when many compounds have to be screened for protein binding. Furthermore it has also been demonstrated that the use of immobilised HSA in high performance liquid chromatography (HPLC) correctly reflects the binding of drugs to free HSA. (Domenici et al., "Immobilized Serum Albumin: Rapid HPLC Probe of Stereoselective Protein Binding Interactions," *Chirality 2*:263-268 (1990) and Domenici et al., "Use of a Human Serum Albumin-Based Chiral Stationary Phase for High-Performance Liquid Chromatography for the Investigation of Protein Binding: Detection of the Allosteric Interaction Between Warfarin and Benzodiazepine Binding Sites," *J. Pharm. Sci., 80,* 164-169 (1991)).

These results encouraged us to investigate the use of biosensor technology for affinity analysis and for ranking of the binding of drug compounds to surface immobilised HSA. The main advantages of biosensor technology (here a surface plasmon resonans, SPR, sensor) are that binding is monitored directly without the use of labels, sample consumption is low and the analysis is rapid and automated. ((Jönsson et al., "Real-Time Biospecific Interaction Analysis. The Integration of Surface Plasmon Resonance Detection, General Biospecific Interface Chemistry and Microfluidics into one Analytical System," *Advances in Biosensors, 2*:291-336 (1992) and Szabo et al., "Surface Plasmon Resonance and its Use in Biomolecular Interaction Analysis," (BIA). *Curr. Opin. Struct. Biol. 5*:699-705 (1995)). In this Example, we present a new method, based on the injection of compounds at a single concentration, for classifying compounds as high, intermediate or low HSA binders by comparing their binding levels to that of selected reference compounds.

### Materials and methods

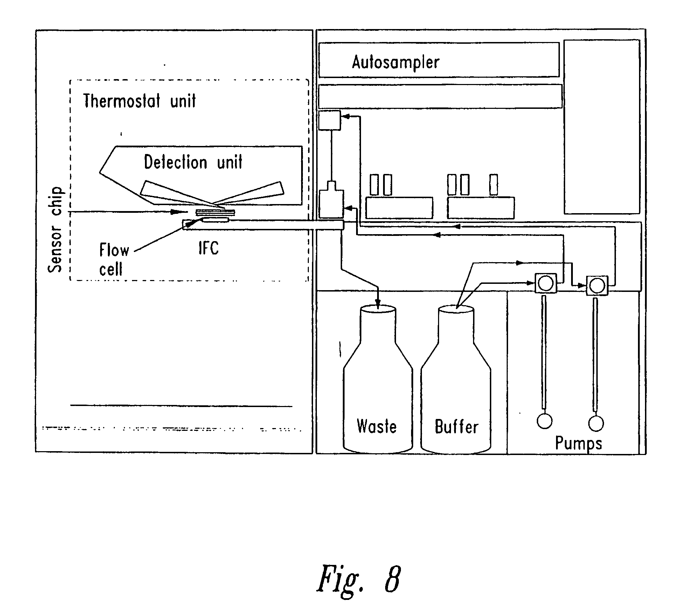

*Instrumentation:* BIACORE 3000 from Biacore AB, Uppsala, Sweden, was used, Figure 8. Interaction analysis was performed at 25°C. *Buffers:* 67 mM isotonic phosphate buffer with 5 % dimethyl sulphoxide (DMSO) pH 7.4 (9.6 g Na₂HPO₄ · 2 H₂O, 1.7 g KH₂PO₄, 4.1 g NaCl to 1 litre) was used as running buffer during the assay. *Immobilisation of HSA:* Sensor chip CM5 was used for analysis. The sensor chip consists of a carboxymethylmodified dextran polymer linked to a gold covered glass support. (Johnsson et al., "Immobilization of Proteins to a Carboxymethyldextran Modified Gold Surface for Biospecific Interaction Analysis in Surface Plasmon Resonance Sensors," *Anal. Biochem., 198*:268-277 (1991).) HSA (Sigma, essentially fatty acid and globulin free, A 3782) at 1 mg/ml was diluted to 15 ug/ml in 10 mM sodium acetate, pH 5.2 and immobilised to the sensor chip, using amine coupling. Running buffer without DMSO was used during immobilisation. The surface was activated by injecting a solution containing 0.2 M N-ethyl-N'-dimethyl aminopropyl carbodiimide (EDC) and 50 mM N-hydroxysuccinimide (NHS) for 7 minutes. HSA was injected during 7 minutes and the surface was then blocked by injecting 1 M ehanolamine at pH 8.5 for 7 minutes. 50 mM NaOH was injected in three 30 seconds pulses to wash offnon covalently bound HSA and to stabilise the baseline. Final immobilisation levels were between 9500 and 11000 RU (Resonance Units). The immobilisation procedure was followed by several washes with buffer to equilibrate the HSA surface in the DMSO running buffer. Unmodified dextran was used as a reference surface. To ensure highest sensitivity the SPR detector response was normalised before running the assay, by calibrating the detector at various light intensities under conditions of total internal reflection (automised procedure). *Compounds:* naproxen, sulphadimethoxin, warfarin, digitoxin, diazepam, naproxen, pyrimethamine, coumarin, salicylic acid, rifampicin, phenytoin, prednisone, quinine, salbutamol, dipyridamole, timolol, acebutolol, atenolol, alprenolol, pindolol, metoprolol and propranolol were from Sigma. Ketanserin was from Fluka. Ritonavir was a gift from Björn Claeson, Medivir. Delavirdine, tolterodine, 5-HM and tipranivir were gifts from Pharmacia & Upjohn. Compounds were selected to: a) cover a wide affinity range towards HSA, b) include binders to major binding sites, c) include different classes of compounds, d) show a range in molecular weight e) if possible be commercially available, see also Table 7. Compounds were diluted from 20 mM DMSO stock solutions to their final concentration in running buffer. Dilution was done in such a way that the pH and the concentrations of both DMSO and buffer substances in samples and running buffer were carefully matched.

### Calibration of DMSO bulk differences between reference and HSA flowcells

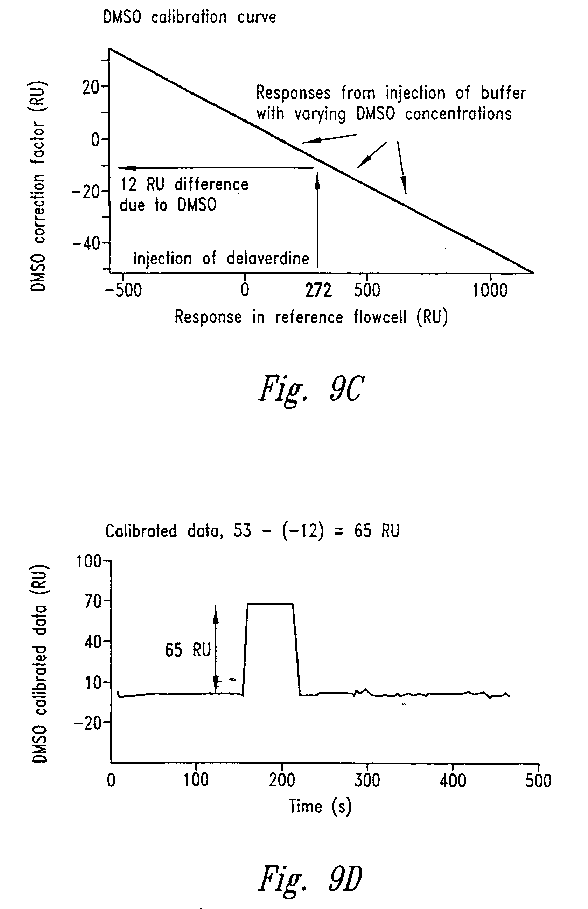

The SPR signal reflects changes in refractive index (RI) at the sensor surface. RI changes as a consequence of binding events close to the sensor surface and are related to the increase of mass on the surface (Stenberg et al., "Quantitative Determination of Surface Concentration of Protein with Surface Plasmon Resonance using Radiolabeled Proteins," *J. Colloid Interface Sci*., *143*:513-526 (1991)). An additional signal is obtained if the injected sample has a RI that differs from that of the running buffer. This signal is referred to as a solvent effect. When the solvent effect is small (in the order of a hundred RU) it can normally be eliminated from the total signal by subtraction of the signal from the reference surface. (Karlsson et al., "Surface Plasmon Resonance Detection and Multi-Spot Sensing for Direct Monitoring of Interactions Involving Low Molecular Weight Analytes and for Determination of Low Affinities," *Anal. Biochem., 228*:274-280 (1995)). However, the introduction of a high refractive index solvent such as DMSO can give rise to large shifts in refractive index during the injection, and mere subtraction of the data from the reference flowcell is no longer sufficient. To correct for these effects a DMSO calibration procedure was adopted (Figure 9c). Buffer solutions with varying concentrations of DMSO (4.5 - 6 %) were injected in sequence over reference and HSA surfaces. This was done at the beginning and end of each experiment, using the same flow rate as for the assay. The responses of the calibration solutions, obtained from the reference surface, covered a range no larger than -500 to +1000 RU relative to the baseline. A DMSO calibration curve was created by plotting the difference in response between HSA and reference flowcells versus the response in the reference flowcell. This curve was used for correcting response levels obtained during sample injection.

*Assay design.* Compounds were injected over the reference and HSA flowcell during one minute at a flow rate of 30 µl/min. Each cycle consisted of a one minute waiting period for monitoring of the baseline stability, a one minute injection of compound, a 35 seconds undisturbed dissociation phase, and a wash of the flow system, except the sensor surface, with a 1:1 mixture of DMSO and water. Compound responses in the reference flowcell were within the range of the DMSO calibration curve, extrapolation of the calibration curve was not used. An injection of running buffer was made between each sample to check for carry over effects. Eight DMSO calibration solutions were injected sequentially, each during 30 seconds, at the beginning and end of each experiment.

*Data evaluation*: Data obtained in the reference flowcell was subtracted from that obtained in the HSA flowcell. Responses from injections of drug compounds and calibration solutions were extracted between report points set at 10 seconds before injection start and 10 seconds before the end of injection. These responses were further corrected for DMSO effects by use of the calibration curves (Figure 9) and the final response values were used in K_{D} determinations and in ranking experiments. To get an estimate of K_{D} and response values at site saturation (Rₘₐₓ), measured response values (R_{eq}) obtained with different concentrations (C) of the compound were fitted to the equation:${\text{R}}_{\text{eq}} {\text{/mw = C×R}}_{\text{max}} {\text{/(C+K}}_{\text{D}} \text{)}$

This equation was derived previously for the general case of an analyte binding to an immobilised partner (Karlsson et al., "Kinetic Analysis of Monoclonal Antibody-Antigen Interactions with a New Biosensor Based Analytical System," *J. Immunol. Methods, 145*:229-240 (1991)) and here provides the affinity and the saturation response for a compound that interacts with a single site of immobilised albumin. To facilitate a comparison of responses obtained with compounds of varying molecular weights R_{eq}/mw *i*.*e*., the steady state response divided with the molecular weight of the compound was used in the fitting procedure instead of R_{eq}. By using this ratio, the saturation level Rₘₐₓ was considered identical for all compounds that bind to a single site of albumin and dose response curves from several compounds were therefore analysed simultaneously with Rₘₐₓ as a global parameter *i*.*e*., common to all compounds and K_{D} as a local parameter *i*.*e*., unique for each compound.

To compare K_{D} calculated by this method with reported levels of % bound the equilibrium equation was used, [HSA] [Drug]/[HSA-drug complex]=K_{D}. A concentration of 0.68 mM of HSA in serum was assumed. With x as the total concentration of compound and p as the part bound (0.68-x×p) × (x-x×p)/x×p = K_{D}. Following an input of p (p is equal to % bound/100) K_{D} values were calculated for total drug concentrations ranging from 0.1 nM to 1 mM. Calculated K_{D} values were largely independent of the total drug concentration. Values for selected compounds are shown in Table 6.

In ranking experiments responses obtained at 80 µM concentration were divided by the molecular weight of the compound to allow comparison of binding levels.

### RESULTS

### Sensorgrams and calibration of solvent effects.

All compounds tested bound reversibly to immobilised HSA. During injection a steady state response was reached within a few seconds. After the end of the injection drug compounds dissociated very rapidly and the signal returned to baseline almost immediately (Figures 9a and 9b). Of the compounds investigated only tipranivir remained bound to the HSA surface for more than two minutes.

To reduce errors in reference subtraction, solvent effects were compensated for by applying a DMSO calibration procedure (Figure 9c). Solvent effects from samples typically ranged from -100 and 500 RU leading to correction factors of -20 to 10 RU. In previous studies remaining solvent related errors have been shown to be low and between +/- 3 RU when solvent effects were as large as 2000 RU (Karlsson et al., "BIACORE Analysis of Drug Target Interactions. Direct and Competitive Binding Assays for Investigation of Interactions between Thrombin and Thrombin Inhibitors," *Anal. Biochem., 278*:1-13 (2000)).

### Activity and stability of immobilised HSA.

The major drug binding sites on HSA remained accessible after immobilisation of HSA to the sensor surface via its amine groups. This was demonstrated by the binding of warfarin (Zatón et al., "Binding of Coumarins to Site I of Human Serum Albumin. Effect of the Fatty Acids," *Chemico-Biol. Int. 97*:169-174 (1995)) to Site I (the warfarin site), naproxen (Cheruvallath et al., "A Quantitative Circular Dichroic Investigation of the Binding of the Enantiomers of Ibuprofen and Naproxen to Human Serum Albumin," *J. Pharm. Biomed. Anal*., *15*:1719-1724 (1997)) to Site II (the benzodiazepin site) and by binding of digitoxin (Kragh-Hansen, U., "Relations between High-Affinity Binding Sites of Markers for Binding Regions on Human Serum Albumin," *Biochem. J., 225*:629-638 (1985)) to its site (Figures 10a-10c). As expected saturation levels were related to the molecular weight of the compounds. Digitoxin with a mw of 765 Da, warfarin with mw 308 Da and naproxen with mw of 230 Da approached saturation levels close to 70, 35 and 15 RU respectively. These values indicated that mainly single sites were occupied at these concentrations with the exception of ritonavir where the response indicated a stoichiometry of more than one ritonavir molecule/HSA molecule (Figure 10d).

In ranking experiments warfarin was used as a positive control of HSA activity. Warfarin was injected early, in the middle and late in each experiment. Reference subtracted and calibrated responses of warfarin from an experiment performed on the day of HSA immobilisation and from an experiment conducted seven days later on the same HSA surface are presented in Table 7. The warfarin response was stable and on this surface response levels ranged from 37.1 to 41.8 RU with a standard deviation of 1.6 RU.

### K_{D} determinations

Dose response curves obtained for warfarin, naproxen, digitoxin, ritonavir, quinine, and rifampicin are shown in Figure 10. For warfarin and naproxen binding appeared monophasic and approached saturation levels at low concentrations. For ritonavir and to some extent digitoxin response levels indicated a biphasic interaction. Quinine and rifampicin gave linear dose response curves over a wide concentration range and it was not possible to estimate saturation levels.

Given the dose response curves in Figure 10, K_{D} analysis was problematic. To obtain an estimate of affinity the analysis was simplified by assuming 1) that HSA binding sites were equally available 2) that only high affinity sites were populated at concentrations below 40 µM and 3) that response levels were normalised by dividing the measured response (RU proportional to g/m²) with the molecular weight (RU/mw proportional to mole/m²). The K_{D} data obtained (from eqn1) with these assumptions showed that compounds with low and medium binding to HSA gave much higher K_{D} values than compounds with high level of binding to HSA and suggested a good correlation between reported % bound and K_{D} but with limited resolution of compounds with binding levels above 97 % bound (Table 8).

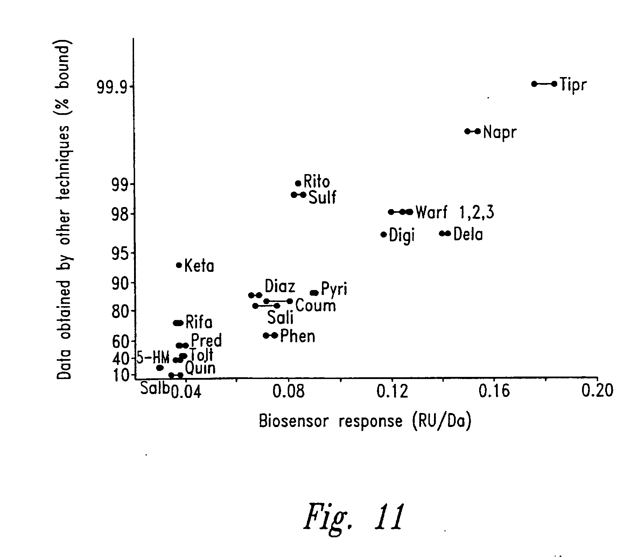

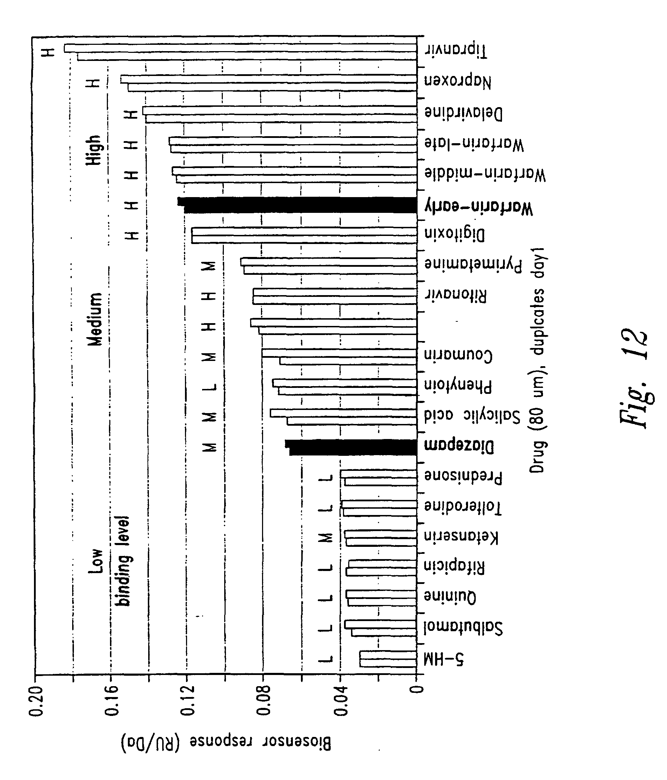

*Ranking of binding levels, comparison of biosensor data with conventional techniques.* For ranking of compounds we used a simpler approach by measuring the degree of binding at a concentration high enough to give a reasonable signal but low enough to avoid misinterpretation of compounds binding to several binding sites. The binding levels of 19 drugs with molecular weights between 138 and 823 Da and with reported HSA binding levels between 8 and 99.9% were studied through duplicate injections of a single concentration (80 µM) of each drug. Identical experiments were run on the day of HSA immobilisation and a week later on the same HSA surface. Figure 11 shows the correlation between binding levels obtained on the day of HSA immobilisation and the reported degree of HSA binding. To increase the resolution of the high affinity compounds, values were put on a logarithmic scale and 2-log₁₀(100-%bound) was plotted as a function of biosensor data. In a screening situation it may be useful to include compounds as markers for medium and high level binders. The data set in Figure 11 can then be re plotted using only the biosensor data (Figure 12). Using diazepam with a reported binding level of 86.1% and warfarin with reported binding of 98% as marker compounds drugs could be divided into Low, Medium and High level binders.

When tested on the same HSA surface a week later the 19 compounds were all classified into the same groups, except for salicylic acid which changed from Medium to Low on the second analysis. The individual ranking within the group of High level binders were the same as on the day of immobilization. Within the Medium and Low group some compounds shifted places but only salicylic acid switched group from Medium to Low.

### Discussion

Early estimates of pharmacokinetic properties can be used to complement data found on the interaction between a compound and its pharmacological target. The combined data can provide a broader understanding of the functional aspects of the compounds and this may be useful for developing them into drug candidates. With respect to protein binding it is of particular interest to identify compounds that are highly bound to plasma proteins. We have developed a screening method to rank the binding of compounds to immobilised HSA, based on biosensor technology.

The use of different methodologies and experimental conditions for determination of HSA binding levels vary considerably and for some compounds used in this Example reported binding data was sometimes conflicting, making a fair correlation study difficult. Examples of variations in reported binding levels included Warfarin 98.0% - 99.6% (Ferrer et al., "The Binding of Benzopyranes to Human Serum Albumin. A Structure-Affinity Study," *J. Protein. Chem.* *17*:115-119 (1998); and Vestberg et al., "High-Affinity Binding of Warfarin, Salicylate and Diazepam to Natural Mutants of Human Serum Albumin Modified in the C-Terminal End,". *Biochem. Pharmacol. 44*:1515-1521 (1992)), Diazepam 86.1% - 98.2% (McNamara et al., "The Protein Binding of Phenytoin, Propranolol, Diazepam and AL01576 (an Aldose Reductase Inhibitor) in Human and Rat Diabetic Serum," *Pharm. Res., 5*:261-265 (1988); and Viani et al., "Binding of Diazepam, Salicylic Acid and Digitoxin to Albumin Isolated from Fetal and Adult Serum," *Dev. Pharmacol. Ther., 17*:100-108 (1991)) and Salicylic Acid 81.7% - 90.5% (Gahramani et al., "Pharmacokinetics and Drug Disposition; Protein Binding of Aspirin and Salicylate Measured by *In Vivo* Ultrafiltration," *Clin. Pharmacol. Ther., 63*:285-295 (1998); and Viani et al., "Binding of Diazepam, Salicylic Acid and Digitoxin to Albumin Isolated from Fetal and Adult Serum," *Dev. Pharmacol. Ther., 17*:100-108 (1991)). For other compounds only one literature value was found, but most likely a similar variation would be found if these compounds were assayed by several methodologies. In spite of this it was possible to correlate biosensor data to HSA binding levels determined with other techniques.

The concentration 80 µM was chosen for the ranking experiments with the purpose of demonstrating ranking of compounds over the entire affinity range. At this concentration the responses obtained for compounds with binding levels lower than 60% were still small, but since many compounds may be of limited solubility and since it was desired to reduce the influence of secondary binding sites, higher concentrations were not used. During preparation of this manuscript we have seen indications that the concentration may be reduced to 10-40 µM, when focusing mainly on identification of high level binders. To discriminate between low affinity binders an alternative approach with an inhibition study with HSA in solution and the drug target immobilised could be used.

By comparing dose response curves and considering experimental errors of a few RU molecular weight adjusted Rₘₐₓ values obtained with compounds binding to different sites were similar. This led to the assumption that the binding sites were equally available. Dramatically different site availability would have resulted in erroneous ranking of binding levels. By using warfarin and diazepam as reference compounds, classification into high (>95%) or low (<80%) level binding was facilitated. Inspection of Figure 12 demonstrates that of the reported high level binders sulfadimethoxin and ritonavir were classified as medium level binders and digitoxin was on the border between the medium and the high level binders. The reported value of 99% bound for ritonavir was obtained in plasma (Vacca, J. and Condra, J., "Clinically Effective HIV-1 Protease Inhibitors," *DDT*, *2*:261-272 (1997)) and it has further been reported (Lazdins et al., "*In Vitro* Effect of Alpha1-Acid Glycoprotein on the Anti-Human Immunodeficiency Virus (HIV) Activity of the Protease Inhibitor CGP 61755: A Comparative Study with other Relevant HIV Protease Inhibitors," *J. Infect. Dis., 175*:1063-1070 (1997)) of a 10-20 fold reduction in plasma binding at the addition of AGP (α1-acid glycoprotein), indicating a significant part of the plasma binding being to AGP. It could therefore be argued that in this respect only sulfadimethoxine was an outlier. Our ranking of Ketanserin as a low level binder is in conflict with the reported value of 93.2 % (Meuldermans et al., "Plasma Protein Binding of Ketanserin and its Distribution in Blood," *Arzneimittelforschung, 38*:794-800 (1988)). Others have reported a lower binding, 90.6% to plasma (Trenk et al., "Pharmacokinetics and Pharmacodynamics of the 5-HT₂ Receptor Antagonist Ketanserin in Man," *J. Cardiovasc. Pharm., 5*:1034-1039 (1983)) which suggests a lower level of binding to HSA, but still the reason for the discrepancy is unknown at this point.

Along with these results, the excellent repeatability of the warfarin data and the stability of the HSA surface makes the biosensor method well suited for use in the prediction of protein binding levels. A particular advantage of the present method was that binding levels obtained with a single injection of the compound could be used for the correlation. One 96 well plate of compounds can be assayed in 24 hours. The method is practical, it provides the necessary information and complicated analysis is avoided. Furthermore ranking experiments can be complemented by competition studies in order to determine site specificity. In such experiments compounds with known site specificity are conjugated with another molecule to increase its mass (and therefore the signal). This marker is mixed with other compounds and a competition for binding site is achieved (Karlsson, R., "Real Time Competitive Kinetic Analysis of Interactions between Low Molecular Weight Ligands in Solution and Surface Immobilised Receptors," *Anal. Biochem., 221*:142-151 (1994)).

Initial attempts to determine K_{D} values from the biosensor data showed that some information on the number of binding sites on HSA could be obtained. To calculate data for low affinity binders when the concentration was well below the K_{D} concentration R_{eq}/mw was used in the fitting procedure. Rₘₐₓ was then used as a global parameter to constrain the fit. This made K_{D} values more consistent than individual calculations of Rₘₐₓ values for each compound. But the analysis was also complex due to difficulties in determining K_{D} for compounds with multiple binding sites. In spite of the complexity in affinity determination, the interpretation of dose response curves for high affinity binders holds considerable potential for obtaining stoichiometric information.

Since SPR technology measures refractive index it is natural that future experiments include investigation of how differences in the refractive indices of compounds affect analysis. Some of the halogens are known to have high refractive index and we have noticed that compounds containing for instance bromine may give higher response values than anticipated from the molecular weight. Thus further studies along this line may be used to fine tune the HSA binding data.

The possibility of studying in similar manner interactions with other plasma proteins such as AGP is ongoing in our laboratory. The first results from these studies are shown in Figure 13. Biosensor data were correlated against published AGP binding levels of tolterodine (Påhlman et al., "Serum Protein Binding of Tolterodine and its Major Metabolites in Humans and Several Animal Species," *Biopharm. Drug Dispos*., *20*:91-99 (1999)), quinine (Wanwimolruk et al., "Plasma Protein Binding of Quinine: Binding to Human Serum Albumin, Alpha 1-Acid Glycoprotein and Plasma from Patients with Malaria," *J. Pharm*. *Pharmacol., 44*:806-811 (1992)), seven β-adrenoceptor blocking drugs (McDonnell et al., "Using Capillary Electrophoresis/Frontal Analysis to Screen Drugs Interacting with Human Serum Proteins," *Electrophoresis, 19*:448-454 (1998); and Belpair et al., "Binding of b-Adrenoceptor Blocking Drugs to Human Serum Albumin, to al-Acid Glycoprotein and to Human Serum," *Eur. J. Clin. Pharmacol., 22*:253-256 (1982)) and dipyridamole (MacGregor, T. and Sardi, E., "In Vitro Protein Behaviour of Dipyridamole," *J. Pharm. Sci., 80*:119-120 (1991)). A good correlation of binding was obtained with the exception of quinine which obtained a higher binding level compared to other techniques. All experimental conditions were the same as for the HSA correlation experiments, except the immobilisation chemistry of AGP (Figure 13). It would therefore be possible to immobilise HSA and AGP in two different flowcells, inject compounds and obtain binding data to both proteins simultaneously.

**TABLE 6**

| COMPOUNDS, MOLECULAR WEIGHTS AND LITERATURE REFERENCES TO HSA BINDING LEVELS | | | | |
|---|---|---|---|---|
| **Compound** | **Molecular weight (Da)** | **Reported binding to HSA (%)** | **Reported binding classified as High, Medium or Low** | **Literature reference of HSA binding** |
| Tipranivir | 603 | 99.9 | H | 8 |
| Naproxen | 230 | 99.7 | H | 9 |
| Ritonavir | 721 | 99 to plasma | H | 10 |
| Sulfadimethoxin | 310 | 98.7 | H | 11 |
| Warfarin | 308 | 98 | H | 12 |
| Delavirdine | 457 | 96.7 | H | 13 |
| Digitoxin | 765 | 96.6 | H | 14 |
| | | | | |
| Ketanserin | 395 | 93.2 | M | 15 |
| Pyrimethamine | 249 | 86.5 | M | 16 |
| Diazepam | 285 | 86.1 | M | 17 |
| Coumarin | 146 | 83.9 | M | 12 |
| Salicylic acid | 138 | 81.7 to plasma | M | 18 |
| | | | | |
| Rifampicin | 823 | 73.3 | L | 19 |
| Phenytoin | 252 | 63.4 | L | 17 |
| Prednisone | 358 | 53.7 | L | 20 |
| Tolterodine | 325 | 42 | L | 21 |
| Quinine | 325 | 34.6 | L | 22 |
| 5-HM | 341 | 23 | L | 21 |
| Salbutamol | 239 | 8 to plasma | L | 23 |

**TABLE 7**

| DATA FROM TWO RANKING EXPERIMENTS ON THE SAME SURFACE, THE FIRST ON THE DAY OF HSA IMMOBILIZATION AND THE SECOND 7 DAYS LATER. WARFARIN (80 µM) WAS INJECTED IN DUPLICATE EARLY, IN THE MIDDLE AND LATE IN EACH ASSAY TO CHECK HSA BINDING ACTIVITY. | | |
|---|---|---|
| **Response (RU) Warfarin day 1** | | **Warfarin day 7** |
| early | 37.1 | 38.4 |
| | 38.2 | 38.8 |
| middle | 38.4 | 41.3 |
| | 38.9 | 41.1 |
| late | 39.1 | 41.8 |
| | 39.3 | 41.8 |
| average: | 38.5 | 40.5 |
| standard deviation: | 0.8 | 1.5 |

**TABLE 8**

| COMPARISON OF LITERATURE DATA OF % BOUND AND THE CORRESPONDING K_{D} FROM THE EQUILIBRIUM EXPRESSION, WITH K_{D} OBTAINED WITH BIOSENSOR ANALYSIS. | | | |
|---|---|---|---|
| Compound | From Literature | | Biosensor Analysis |
| | % bound^{*a*} | K_{D} (µM)^{*b*} | K_{D} (µM) |
| Naproxen | 99.7 | 2 | 26 |
| Ritonavir | 99 | 7 | 21 |
| Warfarin | 98 | 14 | 9 |
| Digitoxin | 97.6 | 17 | 28 |
| Rifampicin | 73.3 | 218 | 195 |
| Quinine | 34.6 | 1290 | 556 |

| | | | |
|---|---|---|---|
| *a* Literature references in Table 6. | | | |
| *b* Calculated from % bound as described in data evaluation | | | |

### EXAMPLE 4

### POSSIBILITY OF IDENTIFYING THE CYTOCHROME P450 ISOZYME INVOLVED IN THE BINDING OF A TEST SUBSTANCE

### Experimental Procedure

The experiments were carried out on a Biacore 3000 instrument using a CM5 sensor chip. The running buffer contained 0.1 M Na/K-phosphate (pH 7.5), 20% glycerol, and 0.05 mM EDTA. The temperature was maintained at 15 °C throughout the experiment.

### Surface preparation

The chip surface was activated by injection of 35 µl of solution containing 5.75 g/l N-hydroxysuccinimide and 37.5 g/l N-ethyl-N'-(3-dimethylaminopropyl)carbodiimide hydrochloride. The proteins were then immobilized by injecting 35 µl of 10 µg/ml solution in a buffer containing 5 mM K-phosphate (pH 6.8), 20% glycerol, 0.05 mM EDTA, and 0.1% Emulgen 913. Unreacted surface groups were then deactivated by injection of 50 µl 0.1 M Tris-HCl (pH 8.0), 0.15 M KCl, and 20% glycerol. The flow rate during surface preparation was 5 µl/min. Surface in the reference cell was not activated.

### Analysis

The substances dissolved in the running buffer at indicated concentrations and injected at a flow rate of 30 µl/min.

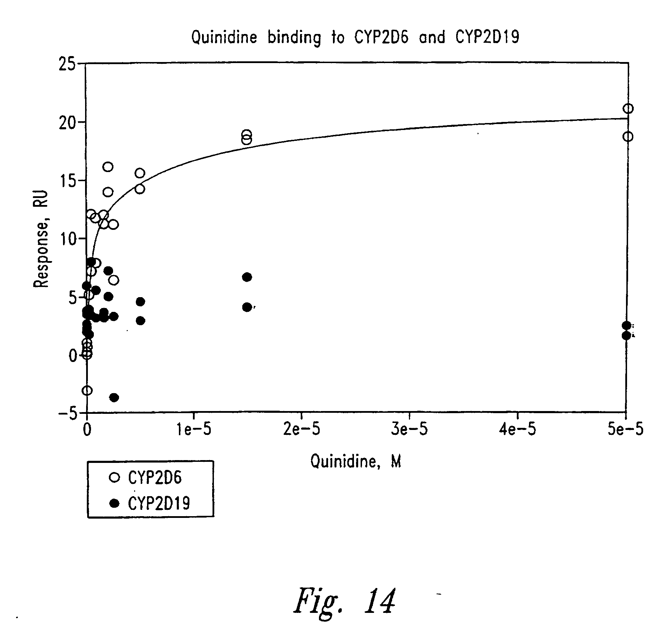

Figure 14. CYP2D6 and CYP2C19 were immobilized in flow cells 3 and 2 to 13000 and 11000 RU, respectively. Flow cell 1 was used as a reference.

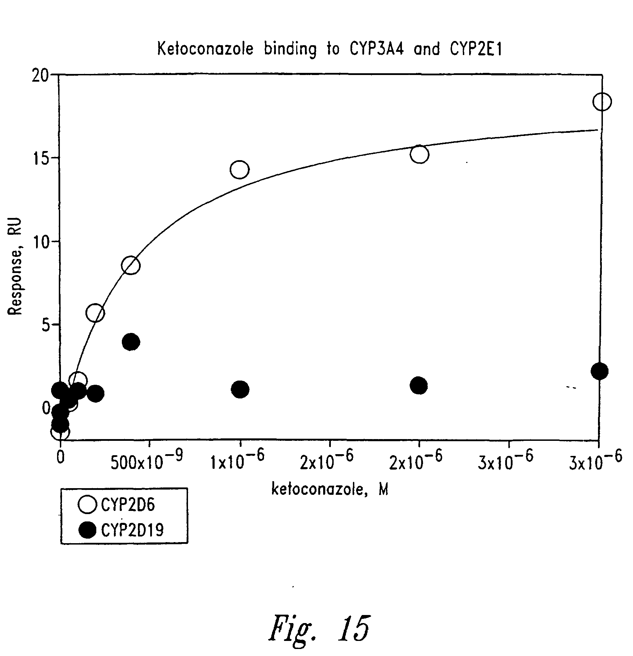

Figure 15. CYP2E1 and CYP3A4 were immobilized in flow cells 2 and 3 to 8500 and 7300 RU, respectively. Flow cell 1 was used as a reference. Cytochromes P450 (CYP) is a group of enzymes involved in the metabolism of many drug substances. The experiments show the possibility of identifying the cytochrome P450 isozyme involved in the binding of a test substance. This is an important pharmacokinetic parameter since it allows a conclusion that (i) the substance has a potential to affect the pharmacokinetics of other drugs known to be metabolized by the same isozyme and (ii) the substance may itself be metabolized by that isozyme. The substances used in the described experiment, ketoconazole and quinidine, are known to be specific inhibitors of CYP3A4 and CYP2D6, respectively. The results obtained demonstrate that BIA assay is able to detect the interaction of these substances with their targets in a specific way.

## Claims

1. A method for assaying a drug candidate with a biosensor having one or more sensing surface-bound biomolecules associated therewith, comprising the steps of:
measuring a binding interaction between the drug candidate and the one or more sensing surface-bound biomolecules of the biosensor to obtain at least one binding interaction parameter of the drug candidate; and
comparing the at least one binding interaction parameter against at least one mathematical expression correlated from binding interaction data associated with known drug compounds having known pharmacokinetic parameters to determine an estimate of at least one pharmacokinetic parameter of the drug candidate.

2. The method of claim 1 wherein the at least one pharmacokinetic parameter is an absorption parameter, a distribution parameter, a metabolism parameter, or an excretion parameter.

3. The method of claim 1 wherein the at least one pharmacokinetic parameter is volume of distribution, total clearance, protein binding, tissue binding, metabolic clearance, renal clearance, hepatic clearance, biliary clearance, intestinal absorption, bioavailability, relative bioavailability, intrinsic clearance, mean residence time, maximum rate of metabolism, Michaelis-Menten constant, partitioning coefficients between tissues and blood or plasma, fraction excreted unchanged in urine, fraction of drug systemically converted to metabolites, elimination rate constant, half-life, or secretion clearance.

4. The method of claim 3 wherein the partitioning coefficients between tissues and blood or plasma are partitioning coefficients associated with the blood brain barrier, blood placenta barrier, blood human milk partitioning, blood adipose tissue partitioning, or blood muscle partitioning.

5. The method of claim 1 wherein an estimate of at least two pharmacokinetic parameters of the drug candidate are determined.

6. The method of claim 1 further comprising determining an estimate of a solubility property of the drug candidate.

7. The method of claim 1 wherein the biosensor utilizes a mass-sensing technique.

8. The method of claim 7 wherein the mass-sensing technique involves surface plasmon resonance.

9. The method of claim 1 wherein the at least one mathematical expression correlated from binding interaction data associated with known drug compounds is a function correlated to a plurality of data points plotted on a Cartesian coordinate system.

10. The method of claim 1 wherein the plurality of sensing surface-bound biomolecules are selected from liposomes, plasma proteins, CYP 450 enzymes, metabolic enzymes, or transport proteins.

11. The method of claim 1 wherein the biosensor utilizes a sensor chip comprising:
a hydrogel coupled to the sensor surface, wherein the hydrogel has a plurality of functional groups, and wherein the one or more sensing surface-bound biomolecules are bonded to the hydrogel.

12. The method of claim 11 wherein the sensor chip further comprises:
a free electron metal that includes a sensor surface, wherein the free electron metal is selected from the group consisting of copper, silver, aluminum and gold.

13. The method of claim 12 wherein the biosensor is capable of detecting surface plasmon resonance associated with the free electron metal.

14. The method of claim 12 wherein the hydrogel is a polysaccharide or a water-swellable organic polymer.

15. The method of claim 14 wherein the polysaccharide is dextran.

16. The method of claim 11 wherein the plurality of functional groups of the hydrogel of the sensor chip include one or more of a hydroxyl, carboxyl, amino, aldehyde, carbonyl, epoxy or vinyl functional group.

17. The method of claim 11 wherein the step of measuring comprises detecting a signal associated with a reflected light beam with respect to time, wherein the-reflected light beam establishes a surface plasmon resonance with the free electron metal.

18. The method of claim 17 wherein the signal associated with the reflected light beam defines a resonance curve of the surface plasmon resonance.

19. The method of claim 17 wherein the signal associated with the reflected light beam defines a reflectance minimum of the surface plasmon resonance.

20. An apparatus for assaying a drug candidate, wherein the apparatus comprises an affinity-based biosensor having one or more sensing surface-bound biomolecules associated therewith and capable of measuring at least one binding interaction parameter of the drug candidate, and a computer memory containing a data structure for comparing the at least one binding interaction parameter against at least one mathematical expression correlated from binding interaction data associated with known drug compounds having known pharmacokinetic parameters to determine an estimate of at least one pharmacokinetic parameter of the drug candidate.

## Patentansprüche

1. Verfahren zum Untersuchen eines Wirkstoffkandidaten mit einem Biosensor, der ein oder mehrere erfassende oberflächengebundene Biomoleküle aufweist, die damit verbunden sind, umfassend die Schritte:
Messen der Bindungswechselwirkung zwischen dem Wirkstoffkandidaten und dem einen oder den mehreren erfassenden oberflächengebundenen Biomolekül(en) des Biosensors zum Erhalten mindestens eines Bindungswechselwirkungsparameters des Wirkstoffkandidaten; und
Vergleichen des mindestens einen Bindungswechselwirkungsparameters mit mindestens einem mathematischen Ausdruck, korrelierend mit den Bindungswechselwirkungsdaten, die mit bekannten Wirkstoffverbindungen verbunden sind, welche bekannte pharmakokinetische Parameter aufweisen, um eine Einschätzung mindestens eines pharmakokinetischen Parameters des Wirkstoffkandidaten vorzunehmen.

2. Verfahren nach Anspruch 1, worin der mindestens eine pharmakokinetische Parameter ein Absorptionsparameter, ein Verteilungsparameter, ein Metabolismusparameter oder ein Exkretionsparameter ist.

3. Verfahren nach Anspruch 1, worin der mindestens eine pharmakokinetische Parameter Verteilungsvolumen, Gesamtclearance, Proteinbindung, Gewebebindung, metabolische Clearance, renale Clearance, hepatische Clearance, biliäre Clearance, intestinale Absorption, Bioverfügbarkeit, relative Bioverfügbarkeit, intrinsische Clearance, mittlere Verweilzeit, Maximalrate des Metabolismus, Michaelis-Menten- Konstante, Verteilungskoeffrzienten zwischen Geweben und Blut oder Plasma, Fraktion, die unverändert in Urin ausgeschieden wird, Wirkstofffraktion, die systemisch in Metaboliten umgewandelt wird, Eliminierungsratenkonstante, Halbwertszeit oder Sekretionsclearance ist.

4. Verfahren nach Anspruch 3, worin die Verteilungskoeffizienten zwischen Geweben und Blut oder Plasma Verteilungskoeffizienten sind, die verbunden sind mit der Blut-Gehirn-Schranke, Blut-Plazenta-Schranke, der Verteilung zwischen Blut und humaner Milch, Verteilung zwischen Blut und Fettgewebe oder Verteilung zwischen Blut und Muskel.

5. Verfahren nach Anspruch 1, worin eine Abschätzung von mindestens zwei pharmakokinetischen Parametern des Wirkstoffkandidaten vorgenommen wird.

6. Verfahren nach Anspruch 1, weiterhin umfassend das Vornehmen einer Abschätzung einer Löslichkeitseigenschaft des Wirkstoffkandidaten.

7. Verfahren nach Anspruch 1, worin der Biosensor eine Masseerfassungstechnik verwendet.

8. Verfahren nach Anspruch 7, worin die Masseerfassungstechnik Oberflächenplasmonresonanztechnik umfasst.

9. Verfahren nach Anspruch 1, worin der mindestens eine mathematische Ausdruck, der korreliert mit Bindungswechselwirkungsdaten, die assoziiert sind mit bekannten Wirkstoffverbindungen, eine Funktion ist, die korreliert ist mit einer Vielzahl von Datenpunkten, die in einem kartesischen Koordinatensystem aufgetragen werden.

10. Verfahren nach Anspruch 1, worin die mehreren erfassenden oberflächengebundenen Biomoleküle ausgewählt werden aus Liposomen, Plasmaproteinen, CYP 450-Enzymen, metabolischen Enzymen oder Transportproteinen.

11. Verfahren nach Anspruch 1, worin der Biosensor einen Sensorchip verwendet, umfassend:
ein Hydrogel, das gekoppelt ist an die Sensoroberfläche, worin das Hydrogel mehrere funktionelle Gruppen aufweist und worin das eine oder die mehreren erfassenden oberflächengebundenen Biomoleküle an das Hydrogel gebunden sind.

12. Verfahren nach Anspruch 11, worin der Sensorchip weiterhin umfasst:
ein Metall mit freien Elektronen, das eine Sensoroberfläche umfasst, worin das Metall mit freien Elektronen ausgewählt ist aus der Gruppe, bestehend aus Kupfer, Silber, Aluminium und Gold.

13. Verfahren nach Anspruch 12, worin der Biosensor zum Nachweisen der Oberflächenplasmonresonanz geeignet ist, die mit dem Metall mit freien Elektronen verbunden ist.

14. Verfahren nach Anspruch 12, worin das Hydrogel ein Polysaccharid oder ein mit Wasser quellbares organisches Polymer ist.

15. Verfahren nach Anspruch 14, worin das Polysaccharid Dextran ist.

16. Verfahren nach Anspruch 11, worin die mehreren funktionellen Gruppen des Hydrogels des Sensorchips eine oder mehrere funktionelle Hydroxyl-, Carboxyl-, Amino-, Aldehyd-, Carbonyl-, Epoxy- oder Vinylgruppen umfassen.

17. Verfahren nach Anspruch 11, worin der Schritt des Messens das Nachweisen eines Signals umfasst, das mit einem reflektierten Lichtstrahl in Bezug auf die Zeit verbunden ist, worin der reflektierte Lichtstrahl eine Oberflächenplasmonresonanz mit dem Metall mit freien Elektronen entwickelt.

18. Verfahren nach Anspruch 17, worin das Signal, das mit dem reflektierten Lichtstrahl verbunden ist, eine Resonanzkurve der Oberflächenplasmonresonanz definiert.

19. Verfahren nach Anspruch 17, worin das Signal, das mit dem reflektierten Lichtstrahl verbunden ist, ein Reflexionsminimum der Oberflächenplasmonresonanz definiert.

20. Vorrichtung zum Untersuchen eines Wirkstoffkandidaten, worin die Vorrichtung einen Biosensor auf Affinitätsbasis, mit einem oder mehreren erfassenden oberflächengebundenen Biomolekülen, die damit verbunden sind, und welcher geeignet ist zum Messen mindestens eines Bindungswechselwirkungsparameters des Wirkstoffkandidaten, und einen Computerspeicher, der eine Datenstruktur aufweist zum Vergleichen des mindestens einen Bindungswechselwirkungsparameters mit mindestens einem mathematischen Ausdruck, korrelierend mit den Bindungswechselwirkungsdaten, die mit bekannten Wirkstoffverbindungen verbunden sind, welche bekannte pharmakokinetische Parameter aufweisen, um eine Einschätzung mindestens eines pharmakokinetischen Parameters des Wirkstoffkandidaten vorzunehmen, umfasst.

## Revendications

1. Méthode pour analyser un médicament candidat avec un biocapteur ayant, à l'état associé à celui-ci, une ou plusieurs biomolécules liées à la surface de captage, comprenant les étapes consistant :
à mesurer l'interaction de liaison entre le médicament candidat et la ou les biomolécules, liées à la surface de captage, du biocapteur pour obtenir au moins un paramètre d'interaction de liaison du médicament candidat ; et
à comparer ledit au moins un paramètre d'interaction de liaison avec au moins une expression mathématique corrélée avec des résultats d'interaction de liaison associés à des composés médicamenteux connus ayant des paramètres pharmacocinétiques connus pour déterminer une estimation d'au moins un paramètre pharmacocinétique du médicament candidat.

2. Méthode suivant la revendication 1, dans laquelle ledit au moins paramètre pharmacocinétique est un paramètre d'absorption, un paramètre de distribution, un paramètre de métabolisme ou un paramètre d'excrétion.

3. Méthode suivant la revendication 1, dans laquelle ledit au moins un paramètre pharmacocinétique est le volume de distribution, la clairance totale, la liaison aux protéines, la liaison à un tissu, la clairance métabolique, la clairance rénale, la clairance hépatique, la clairance biliaire, l'absorption intestinale, la biodisponibilité, la biodisponibilité relative, la clairance intrinsèque, le temps de séjour moyen, la vitesse maximale de métabolisme, la constante de Michaelis-Menten, les coefficients de partage entre les tissus et le sang ou le plasma, la fraction excrétée inchangée dans l'urine, la fraction d'un médicament ayant subi une conversion systémique en métabolites, la constante de vitesse d'élimination, la demi-vie ou la clairance de sécrétion.

4. Méthode suivant la revendication 3, dans laquelle les coefficients de partage entre les tissus et le sang ou le plasma sont les coefficients de partage associés à la barrière hématoencéphalique, la barrière hématoplacentaire, le partage entre le sang et le lait humain, le partage entre le sang et le tissu adipeux ou le partage entre le sang et le muscle.

5. Méthode suivant la revendication 1, dans laquelle une estimation d'au moins deux paramètres pharmacocinétiques du médicament candidat est déterminée.

6. Méthode suivant la revendication 1, comprenant en outre la détermination d'une estimation d'une propriété de solubilité du médicament candidat.

7. Méthode suivant la revendication 1, dans laquelle le biocapteur utilise une technique de captage de masse.

8. Méthode suivant la revendication 7, dans laquelle la technique de captage de masse implique la résonance de plasmon de surface.

9. Méthode suivant la revendication 1, dans laquelle ladite au moins une expression mathématique corrélée aux résultats d'interaction de liaison associés à des composés médicamenteux connus est une fonction corrélée à une pluralité de points de résultats représentés graphiquement sur un système en coordonnées cartésiennes.

10. Méthode suivant la revendication 1, dans laquelle la pluralité de biomolécules liées à la surface de captage est choisie entre des liposomes, des protéines plasmatiques, des enzymes CYP 450, des enzymes métaboliques et des protéines de transport.

11. Méthode suivant la revendication 1, dans laquelle le biocapteur utilise une puce de captage comprenant :
un hydrogel couplé à la surface du capteur, l'hydrogel ayant une pluralité de groupes fonctionnels, et la ou les biomolécules liées à la surface de captage étant liées à l'hydrogel.

12. Méthode suivant la revendication 11, dans laquelle la puce de captage comprend en outre :
un métal à électrons libres qui comprend une surface de captage, ledit métal à électrons libres étant choisi dans le groupe consistant en cuivre, argent, aluminium et or.

13. Méthode suivant la revendication 12, dans laquelle le biocapteur est capable de détecter la résonance de plasmon de surface associée au métal à électrons libres.

14. Méthode suivant la revendication 12, dans laquelle l'hydrogel est un polysaccharide ou un polymère organique pouvant gonfler dans l'eau.

15. Méthode suivant la revendication 14, dans laquelle le polysaccharide est le dextrane.

16. Méthode suivant la revendication 11, dans laquelle la pluralité de groupes fonctionnels de l'hydrogel de la puce de captage comprend un ou plusieurs des groupes consistant en les groupes fonctionnels hydroxyle, carboxyle, amino, aldéhyde, carbonyle, époxy et vinyle.

17. Méthode suivant la revendication 11, dans laquelle l'étape de mesure comprend la détection d'un signal associé à un faisceau lumineux réfléchi en fonction du temps, le faisceau lumineux réfléchi établissant une résonance de plasmon de surface avec le métal à électrons libres.

18. Méthode suivant la revendication 17, dans laquelle le signal associé au faisceau lumineux réfléchi définit une courbe de résonance de la résonance de plasmon de surface.

19. Méthode suivant la revendication 17, dans laquelle le signal associé au faisceau lumineux réfléchi définit un minimum de réflectance de la résonance de plasmon de surface.

20. Appareil pour l'analyse d'un médicament candidat, ledit appareil comprenant un biocapteur basé sur l'affinité ayant, à l'état associé à celui-ci, une ou plusieurs biomolécules liées à une surface de captage, et capable de mesurer au moins un paramètre d'interaction de liaison du médicament candidat, et une mémoire informatique contenant une structure de données pour comparer ledit au moins un paramètre d'interaction de liaison avec au moins une expression mathématique corrélée à partir des résultats d'interaction associés à des composés médicamenteux connus ayant des paramètres pharmacocinétiques connus pour déterminer une estimation d'au moins un paramètre pharmacocinétique du médicament candidat.
